(19) Europäisches Patentamt / European Patent Office / Office européen des brevets

(11) EP 3 504 179 B1

(12) FASCICULE DE BREVET EUROPEEN

(45) Date de publication et mention de la délivrance du brevet: **16.03.2022 Bulletin 2022/11**

(21) Numéro de dépôt: **17768868.6**

(22) Date de dépôt: **24.08.2017**

(51) Classification Internationale des Brevets (IPC):
***C07C 67/08*** (2006.01) ***C08G 63/66*** (2006.01)
***C08G 63/668*** (2006.01) ***C08G 18/42*** (2006.01)

(52) Classification Coopérative des Brevets (CPC):
(C-Sets disponibles)
**C08G 18/42; C07C 69/40; C07C 69/44; C08G 18/4244; C08G 63/668;** C08G 2110/0008; C08G 2110/0016; C08G 2110/0025 (Cont.)

(86) Numéro de dépôt international:
**PCT/IB2017/055107**

(87) Numéro de publication internationale:
**WO 2018/037371 (01.03.2018 Gazette 2018/09)**

(54) **METHODE DE PRODUCTION DE POLYOL POLYESTERS ET LEUR UTILISATION DANS LE POLYURETHANE**

VERFAHREN ZUR HERSTELLUNG VON POLYETHERPOLYOLEN UND VERWENDUNG DAVON BEI POLYURETHAN

METHOD FOR PRODUCING POLYESTER POLYOLS AND USE THEREOF IN POLYURETHANE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **24.08.2016 FR 1601253**

(43) Date de publication de la demande:
**03.07.2019 Bulletin 2019/27**

(73) Titulaires:
- **Tereos Starch & Sweeteners Belgium 9300 Aalst (BE)**
- **Centre National de la Recherche Scientifique 75794 Paris Cedex 16 (FR)**
- **Société Soprema SAS 67025 Strasbourg (FR)**
- **Université de Strasbourg 67081 Strasbourg Cedex (FR)**

(72) Inventeurs:
- **BINDSCHEDLER, Pierre Etienne 67025 Strasbourg Cedex (FR)**
- **SARBU, Alexandru 67025 Strasbourg Cedex (FR)**
- **LAURICHESSE, Stephanie 67025 Strasbourg Cedex (FR)**
- **PERRIN, Remi 67025 Strasbourg Cedex (FR)**
- **FURTWENGLER, Pierre 75794 Paris Cedex 16 (RE)**
- **AVÉROUS, Luc 75794 Paris Cedex 16 (FR)**
- **REDL, Andreas 77230 Moussy-le-Vieux (FR)**

(74) Mandataire: **Icosa 83 avenue Denfert-Rochereau 75014 Paris (FR)**

(56) Documents cités:
**US-A- 2 863 855 US-A- 4 001 180**
**US-A- 4 404 295**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 504 179 B1

(52) Classification Coopérative des Brevets (CPC):
(Cont.)

C-Sets
**C07C 67/08, C07C 69/34**

## Description

### Domaine technique

La présente invention concerne un procédé d'obtention de polyol polyester, les polyols polyester obtenus par la mise en œuvre d'un tel procédé, l'utilisation de tels polyols polyesters dans la fabrication des mousses, adhésifs, revêtements ou élastomères de polyuréthane ou de polyisocyanurate.

### Résumé de l'invention

La présente invention est définie conformément aux revendications annexes.

### Arrière-plan technique

De nos jours la recherche de produits d'origine renouvelable pouvant se substituer aux produits pétro-sourcés constitue une stratégie d'avenir pour réduire notre dépendance aux ressources fossiles. Les polyuréthanes constituent une famille importante de polymères, très demandeuse de composés d'origine biosourcés. Le secteur de l'industrie du bâtiment recherche des matériaux biosourcés et durables, notamment dans le domaine des mousses qui peuvent être utilisées pour l'isolation thermique et/ou acoustique dans le bâtiment. Les utilisations des polyuréthanes dans ce secteur se font essentiellement sous forme de mousses rigides, polyuréthane (PUR) et polyisocyanurates (PIR).

Les matériaux polyuréthane (mousses rigides et souples, élastomères, revêtement, adhésif...) reposent sur la réaction de polyaddition entre un polyisocyanate et un polyol. Il apparaît donc nécessaire de travailler à l'élaboration de nouveaux matériaux toujours plus compétitifs tels que des polyols d'origine renouvelable constitués de synthons issus de la biomasse dont la production à haut tonnage est possible.

De nos jours, le sorbitol est essentiellement utilisé comme « starter » dans l'élaboration de polyols polyéther en utilisant une chimie dite d'alcoxylation qui est un procédé relativement dangereux notamment du fait des réactifs utilisés. L'alcoxylation est une méthode industrielle de chimie à haute température et sous pression faisant réagir des oxydes d'alkylène sur un starter (sorbitol par exemple) en présence d'un catalyseur créant ainsi des chaînes polyéthers. Ce procédé couteux et complexe utilise en outre, des réactifs dangereux.

Le second enjeu repose également sur la maîtrise de la modification chimique du sorbitol puisque l'alcoxylation touche toutes les fonctions hydroxyles du sorbitol créant des polyols polyéthers à haute fonctionnalité (typiquement 6). Or, le marché des mousses polyuréthanes est un des marchés consommateurs de polyols polyéthers et de plus en plus de polyols polyesters. En outre, dans le domaine de l'isolation thermique, on observe une transition du marché des mousses polyuréthanes vers les mousses polyisocyanurates plus performantes mais demandeuses de nouvelles familles de polyols avec des fonctionnalités plus faibles que celles demandées lors de l'élaboration d'une mousse polyuréthane.

Le sorbitol ($C_6H_{14}O_6$,182.17 g/mol) aussi appelé D-glucitol (N° CAS:50-70-4) ou (2R,3R,4R,5S)-hexane-1,2,3,4,5,6 hexol est un sucre réduit issu de l'hydrogénation de glucose ; il est également naturellement présent dans plusieurs fruits. De nombreuses modifications chimiques sont possibles sur le sorbitol. Le sorbitol se présente sous forme solide et cristalline à 23°C. Cependant, pour l'utilisation du sorbitol dans des polyols destinés à l'élaboration de mousses polyuréthanes, les modifications chimiques principales sont obtenues par réaction d'oxypropylation (Ionescu, *Chemistry and Technology of Polyols for Polyurethanes)* ou de polycondensation.

L'oxypropylation est largement décrite dans la littérature depuis plus de 65 ans (US2605233). Il s'agit d'une réaction exothermique qui a lieu en réacteur fermé sous pression et catalysée à chaud. Elle consiste en une ouverture de cycle pouvant être effectuée aussi bien sous catalyse acide que catalyse basique, cette dernière étant majoritairement utilisée (schéma 1). La réaction consiste en l'ajout successif de molécules d'oxyde de propylène ($C_3H_6O$) ou d'oxyde de butylène ($C_4H_8O$) sur une molécule de départ, portant des fonctions hydroxyles, pouvant être un sucre (US20140200327) ou un oligo-polyol ayant une fonctionnalité comprise entre 2 et 6. Pour amorcer la réaction, le milieu réactionnel est chauffé à des températures comprises entre 100°C et 200°C, une fois la réaction amorcée, une élévation violente de température et de pression est observée.

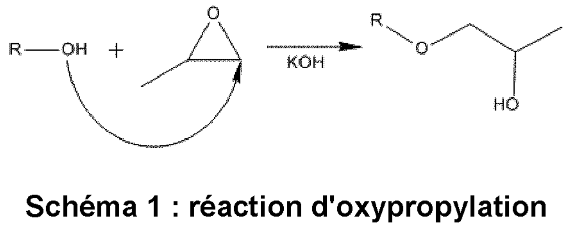


**Schéma 1 : réaction d'oxypropylation**

La première description d'une réaction d'oxypropylation sur le sorbitol a été décrite en 1958 par Joseph E. Wilson et Raymond H. Fowler (Rigid Urethanes Foams Based on Sorbitol Derivatives Science-1958-WILSON-1343*)* conduisant à des polyols ayant une masse molaire (Mn) comprise entre 760 et 4830 g.$mol^{-1}$ et un nombre d'hydroxyles compris entre 70 et 440 mg KOH/g. Le nombre d'hydroxyles étant défini comme une valeur quantitative du nombre de groupements hydroxyles pouvant réagir avec un polyisocyanate exprimé en milligrammes d'hydroxyde de potassium équivalent par gramme de produit. De nombreux travaux académiques ou industriels décrivent des techniques d'oxypropylation, notamment celles mettant en jeu le sorbitol comme molécule de départ.

La polycondensation décrite pour la première fois par Carothers *(*Wallace H. Carothers, J. Am. Chem Soc. 51, no. 8 (1929): 2548-59 and 2560-70*)* est une réaction entre deux molécules portant chacune une fonction réactive et dont le produit de la réaction est composé de l'association des deux molécules et de la formation d'une petite molécule. Dans le cas des polyesters, les fonctions réactives sont une fonction alcool typiquement, les alcools primaires et une fonction acide. Dans ce cas, la petite molécule issue de la réaction est une molécule d'eau (Schéma 2).

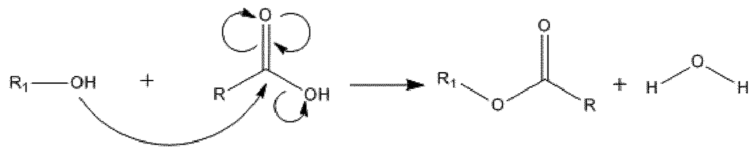

**Schéma 2 : Réaction entre un alcool et un acide**

Pour obtenir un polyester à proprement dit, les monomères utilisés doivent au minimum être des diacides et des diols afin que la réaction soit pérenne. Afin de déplacer l'équilibre de la réaction dans le sens du produit (polyester) et obtenir des masses moléculaires plus élevées, la molécule d'eau créée à chaque addition est éliminée. Afin d'augmenter les masses molaires des polymères obtenus, la réaction peut être catalysée. Les deux grandes familles de catalyse sont la catalyse protonique et les catalyses par les composés organométalliques auquel vient s'ajouter la catalyse enzymatique qui est de plus en plus utilisée.

Un exemple de réaction de polycondensation catalysée par de l'acétate de zinc est décrite par *Abhijeet Anand et al. (*Abhijeet Anand et al Progress in Organic Coatings 74, no. 4 (August 2012): 764-67), entre le D-sorbitol, l'anhydride d'acide 1,2,3,6 tétrahydrophtalique (THPA), le diéthylène glycol (DEG) et l'acide adipique. La réaction décrite et effectuée en deux étapes, une première à 145-150°C entre le sorbitol, le THPA, le DEG et l'acétate de zinc pendant 1h puis la température est élevée à 200°C avec l'ajout de l'acide adipique pendant 5-6h. Sont alors obtenus des polyols polyester avec une masse molaire de 1074 g.$mol^{-1}$. Néanmoins, par analyse infrarouge un pic caractéristique d'une liaison cyclique de type éther à 1068 $cm^{-1}$ attribué à la cyclisation du sorbitol a été observé. Une telle cyclisation est liée à la température élevée de réaction. Cette cyclisation du sorbitol le rend indisponible pour la réaction et réduit donc le rendement de la réaction.

Une autre polymérisation de type polycondensation a été étudiée par *L. Gustini et al. (*L. Gustini et al., " European Polymer Journal 67 (June 2015): 459-75). Il s'agit de polycondensation catalysée par voie enzymatique avec l'enzyme candida antartica lipase B (CALB). L'avantage de cette enzyme est sa sélectivité pour estérifier en priorité les hydroxyles primaires face aux secondaires et ainsi éviter tout type de réticulation lors de la synthèse de polymère. Néanmoins, la quantité de sorbitol incorporé est très faible et peu pertinente puisque l'enzyme CALB ne fonctionne bien qu'avec des monomères apolaires (le sorbitol est au contraire très polaire). Pour contrer cela les auteurs compensent l'apport en sorbitol par une grande quantité de monomère apolaire (1.8 octanediol). Enfin, le procédé propose des synthèses relativement complexes et de très longues durées (en général plus de 24h). L'ensemble de ces limites en font un procédé difficilement exploitable industriellement.

Enfin, un dernier type de polymérisation similaire est proposé dans la demande de brevet FR 2 987 840 déposée par la société NOVANCE décrivant la polycondensation entre un acide gras et le sorbitol ou le glycérol pour obtenir des polyesters. Ce procédé est également complexe puisqu'une étape d'alcoxylation précède l'étape de polycondensation. En outre, des températures appliquées sont de 220°C pour des temps réactionnels relativement longs de 15 à 20h.

Le document US 4 404 295 A décrit des compositions de polyester polyols utilisés pour préparer des mousses de polyuréthane (colonne 1, l.10 15). L'exemple 3 décrit la préparation d'un polyester polyol à partir d'acide adipique, d'anhydride phtalique, de diéthylène glycol et de glycérol, par un procédé en une seule étape.

Il y a donc un besoin d'un procédé simple, écologique (sans solvant, utilisant des produits biosourcés, à pression atmosphérique) et permettant l'obtention à haut rendement de polyesters utilisables notamment dans des polymères de polyuréthane ayant des propriétés aussi bonnes que celles des polymères obtenus à partir de produits dérivés du pétrole.

**Description détaillée de l'invention**

La présente divulgation concerne un **polyol polyester** obtenu par une première polycondensation (a) d'un sucre alcool Z en C3 à C8 et de deux diacides Y et Y' identiques ou différents en C4 à C36 et d'une seconde polycondensation (b) du produit obtenu en (a) avec deux diols X et X' identiques ou différents en C2 à C12.

L'invention concerne un **polyol polyester** de formule générale **Rx-Ry-Z-Ry'-Rx'** dans laquelle

- **Z** est un sucre alcool en C3 à C8, préférentiellement en C4 à C7, typiquement en C5, C6,
- **Ry** et **Ry'** sont des diesters de formule $-OOC-C_n-COO-$ avec n compris entre 2 et 34, préférentiellement, entre 3 et 22, typiquement entre 4 et 10,
- **Rx** et **Rx'** sont des monoalcools identiques ou différents en C2 à C12, préférentiellement en C3 à C8, typiquement en C4.

Le terme « polyol polyester » fait référence à des molécules comprenant des groupements hydroxyles (diols ou sucres alcools) liés entre eux par des liaisons esters. Ainsi, dans le polyol polyester selon l'invention, les molécules X, Y, Z, Y'et X' sont liées entre elles par des liaisons esters. Typiquement, les diols X et X' et le sucre alcool Z sont liés aux deux diacides Y et Y' par des liaisons esters formées chacune entre une fonction acide de Y ou de Y' et une fonction hydroxyle primaire de Z, X ou X'. Avantageusement, le polyol polyester est à pH neutre, typiquement, lorsqu'il est obtenu par deux polycondensations successives suivies d'une étape de neutralisation (par exemple à la potasse ou à la soude).

Le polyol polyester selon l'invention présente avantageusement, la formule chimique générale $C_aH_bO_c$ avec $22 \le a \le 42$, $38 \le b \le 78$, $14 \le c \le 22$.

Typiquement, le polyol polyester selon l'invention présente une masse moléculaire comprise entre 350 g/mol et 2000 g/mol, préférentiellement entre 420 g/mol et 1800 g/mol et plus préférentiellement, entre 450 et 1700 g/mol. Selon l'invention, la masse molaire du polyol polyester peut être déterminée par différentes méthodes telles que la chromatographie d'exclusion stérique.

Avantageusement, le polyol polyester présente un indice hydroxyle de 300 à 900 mg KOH/g. L'indice hydroxyle (IOH) peut être calculé avec la formule suivante :

$$IOH = \text{fonctionnalité du polyol polyester} \times 56109.37 / \text{Masse molaire du polyol polyester}$$

L'indice hydroxyle correspond au nombre de mg de KOH nécessaire pour déprotoner l'ensemble des groupements hydroxyle présents dans un gramme de polyol. L'indice d'hydroxyle peut être déterminé par dosage inverse utilisant de la potasse, par exemple selon la norme ASTM 4274-99 dans laquelle la titration colorimétrique est remplacée par une titration pH-métrique.

Par « sucre alcool » ou « polyol » on entend une forme hydrogénée de monosaccharide dont le groupe carbonyle (aldéhyde ou cétone) a été réduit en un hydroxyle primaire ou secondaire. Typiquement, le sucre alcool est choisi parmi le glycérol, l'éthylène glycol, le sorbitol, l'érythritol, le xylitol, l'arabitol, le ribitol, le dulcitol, le mannitol et le volémitol.

Par « diacide » on entend, une chaîne carbonée comprenant deux groupements acide. Selon l'invention, le polyol polyester comprend deux molécules Y et Y' de diacide. Ces molécules peuvent être identiques ou différentes en C4 à C36, préférentiellement C4 à C24. Typiquement, les deux molécules de diacide sont indépendamment choisies parmi l'acide butanedioïque (Acide succinique), l'acide pentanedioïque (Acide glutarique), l'acide hexanedioïque (Acide adipique), l'acide heptanedioïque (Acide pimélique), l'acide octanedioïque (Acide subérique), l'acide nonanedioïque (Acide azélaïque), l'acide décanedioïque (Acide sébacique), l'acide undécanedioïque, l'acide dodécanedioïque, l'acide tridécanedioïque (Acide brassylique), l'acide tétradécanedioïque, l'acide pentadécanedioïque, l'acide hexadécanedioïque, dimères d'acides gras ayant jusqu'à 36 carbones (C36) ou leur mélange. Typiquement, Y et Y' sont des diacides en C5 à C16 ou C6 à C12. Avantageusement, les molécules de diacide préférées sont indépendamment choisies parmi l'acide adipique et l'acide succinique.

Par « diol » on entend, une chaîne carbonée comprenant deux groupements alcool. Selon l'invention, le polyol polyester comprend deux molécules X et X' de diols identiques ou différentes. Typiquement, les molécules de diol sont indépendamment choisies parmi le 1,2-éthanediol, le 1,3-propanediol, le 1,4-butanediol, le 1,6-hexanediol, le 1,8-octanediol, le 1,10-décanediol, le 1,12-dodécanediol et leur mélange.

Avantageusement, le polyol polyester selon l'invention est choisi parmi le bis(1,2-éthanediol)-sorbitol diadipate, le bis(1,3 propanediol)-sorbitol diadipate, le bis(1,4-butanediol)-sorbitol diadipate, le bis(1,4-butanediol)-sorbitol diadipate modifié avec du glycérol, le bis(1,6-hexanediol)-sorbitol diadipate, le bis(1,8-octanediol)-sorbitol diadipate, le bis(1,10-décanediol)-sorbitol diadipate, le bis(1,12-dodécanediol)-sorbitol diadipate, le bis(1,4-butanediol)-sorbitol disuccinate et le sorbitol-diadipate-sorbitol. Préférentiellement, ledit polyolpolyester est choisi parmi le bis(1,8-octanediol)-sorbitol diadipate, le bis(1,10-décanediol)-sorbitol diadipate et le bis(1,4-butanediol)-sorbitol diadipate.

L'invention concerne également un procédé d'obtention d'un polyol polyester selon l'invention comprenant les étapes suivantes :

a) une étape de polycondensation à une température comprise entre 110 et 200°C, préférentiellement, 120 à 180°C, plus préférentiellement, 130 et 170°C, typiquement 150°C, avantageusement durant 5 à 10 heures:

i. d'un sucre alcool Z en C3 à C8, préférentiellement en C4 à C7, avantageusement en C5-C6, typiquement choisi parmi le glycérol, le sorbitol, l'érythritol, le xylitol, l'arabitol, le ribitol, le dulcitol, le mannitol et le volémitol,
ii. de deux diacides Y et Y' identiques ou différents en C4 à C36, préférentiellement en C5 à C24,
iii. de deux diols X et X' identiques ou différents en C2 à C12, préférentiellement en C3 à C8, typiquement en C4 avantageusement, indépendamment choisi parmi le 1,2-éthanediol, le 1,3-propanediol, le 1,4-butanediol, le 1,6-hexanediol, le 1,8-octanediol, le 1,10-décanediol, le 1,12-dodécanediol, le 1,4-butanediol et leur mélange,

b) optionnellement, une étape de neutralisation des fonctions acides libres de sorte à ramener le polyol polyester à un pH neutre (pH=7), par exemple, par une base typiquement, une base forte telle que du bicarbonate de la soude ou de la potasse, ou par une base faible telle que le carbonate de sodium, le bicarbonate de sodium, le carbonate de potassium ou un mono bi ou trialcool en C4 à C8, tel que de l'hexanol ; préférentiellement l'étape de neutralisation est effectuée par ajout de carbonate de potassium ou de la potasse.

Avantageusement, lors de l'étape de polycondensation, les diols X et X' et le sucre alcool Z sont à un ratio molaire (X+X')/Z compris entre 1 et 3, préférentiellement entre 1,5 et 2,5 encore plus préférentiellement compris entre 1,8 et 2,2.

Typiquement lors de l'étape de polycondensation, les diacides Y et Y' et le sucre alcool Z sont à un ratio molaire (Y+Y')/Z compris entre 1 et 3, préférentiellement entre 1,5 et 2,5 encore plus préférentiellement compris entre 1,8 et 2,2.

Selon un mode de réalisation, lors de l'étape de polycondensation, les diols X et X' et les diacides Y et Y' sont à un ratio molaire (X+X')/(Y+Y') compris entre 0,5 et 2, préférentiellement entre 0,7 et 1,5 encore plus préférentiellement compris entre 0,8 et 1,2.

Dans le procédé selon l'invention, l'étape de polycondensation comprend une première polycondensation (a) du sucre alcool Z et des diacides Y et Y' et une seconde polycondensation (b) du produit obtenu en (a) avec les diols X et X'. Cette polycondensation en deux étapes permet l'obtention du polyol polyester avec cette structure symétrique. Typiquement, les diacides Y et Y' sont identiques et/ou les diols X et X' sont identiques.

Selon un mode de réalisation, le sucre alcool Z est mélangé avec la ou les molécules de diacide Y et Y' puis incubé durant plus d'une heure, plus préférentiellement entre 2 et 5 heures, encore plus préférentiellement entre 2,5 et 4h, typiquement durant 3 heures. La ou les molécules de diol X et X' sont ajoutées dans un second temps au mélange puis incubées durant plus de 4 heures, préférentiellement, entre 5 et 10 heures, typiquement, entre 5,5 et 7h. De manière préférentielle, l'étape de polycondensation est effectuée sous vide.

Avantageusement, lors de l'étape de polycondensation, les molécules de diacide Y et Y' réagissent avec les alcools primaires des molécules de sucre alcool Z puis des diols X et X'. Les molécules d'eau issues de la réaction sont récupérées en vue de leur élimination.

La présente divulgation concerne en outre un **polymère** comprenant le polyol polyester selon l'invention. Selon l'invention, ledit polymère est un polyuréthane et/ou un polyisocyanurate. Avantageusement, le polymère selon l'invention présente un faible taux de polymérisation de 2 à 10, on parle d'un pré-polymère. Un tel pré-polymère peut être alors présent dans une composition destinée à former une mousse, un adhésif, un élastomère ou un revêtement. Selon un mode de réalisation, le polymère selon l'invention présente un haut taux de polymérisation typiquement, supérieur à 10, un tel polymère peut être alors présent dans une mousse, un adhésif, un élastomère ou un revêtement.

Avantageusement, le polymère selon l'invention comprend une masse molaire supérieure à 1000 g/mol.

Selon un mode de réalisation, le polymère selon l'invention présente une masse molaire comprise entre 1000 g/mol et 10 000 g/mol, préférentiellement entre 2000 g/mol et 7000 g/mol. Typiquement, le polymère est un pré-polymère. Un tel pré-polymère lors d'une étape de polymérisation donnera un polymère non-réticulé ou réticulé.

Selon un autre mode de réalisation, le polymère selon l'invention présente une masse molaire comprise entre $1.10^4$ et $1,7.10^6$ g/mol. Typiquement, le polymère est un polymère non réticulé.

Selon un autre mode de réalisation, le polymère selon l'invention présente une masse molaire supérieure à $1,7.10^6$ g/mol. Typiquement, le polymère est un polymère réticulé.

Par « polyuréthane », on entend un polymère comprenant des fonctions uréthanes autrement dit, un polymère d'uréthane. Ces polymères résultent essentiellement de la réaction de polyols notamment du polyol polyester de l'invention et de polyisocyanates. Ces polymères sont généralement obtenus à partir de formulations présentant un index de 100 à 150 correspondant à un rapport NCO/OH compris entre 1 et 1,5.

Par « polyisocyanurate », on entend les polymères résultant de la réaction de polyols notamment du polyol polyester de l'invention et de polyisocyanates, qui contiennent, en plus des liaisons d'uréthane, d'autres types de groupes fonctionnels, en particulier des anneaux triisocyanuriques formés par trimérisation des polyisocyanates. Ces polymères, normalement appelés aussi les polyuréthanes modifiés, sont généralement obtenus à partir de formulations présentant un index 150-450, soit un rapport NCO/OH compris entre 1,5 et 4,5.

Par ratio NCO/OH on entend, au sens de la présente invention, le rapport entre le nombre de fonctions NCO du polyisocyanate et le nombre de fonctions OH du sucre alcool du diol et de tout autre composant comportant de groupements OH (eau, solvants). Le ratio NCO/OH est calculé avec la formule suivante :

$$\text{Ratio NCO / OH} = M_{exp}Pi \times ME\ Pi / M_{exp}\ SAI \times ME\ SAI$$

où :

- $M_{exp}Pi$ est la masse du polyisocyanate ;
- $M_{exp}SAI$ est la masse du sucre alcool ;
- ME SAI est la masse équivalente du sucre alcool et correspond au ratio entre la masse molaire du sucre alcool et la fonctionnalité du sucre alcool ;
- MEPi est la masse équivalente du polyisocyanate et correspond au ratio entre la masse molaire du polyisocyanate et la fonctionnalité du polyisocyanate.

L'invention concerne également une **composition** comprenant ledit polyol polyester selon l'invention ou ledit polymère selon l'invention. Avantageusement, ladite composition est une mousse, un élastomère, un adhésif, un revêtement ou une composition permettant l'obtention de l'un quelconque des mousses, élastomères, adhésifs et revêtements après polymérisation.

Typiquement, la composition selon l'invention comprenant ledit polyol polyester selon l'invention ou ledit polymère selon l'invention notamment le prépolymère, comprend en outre, un catalyseur de réaction, un polyisocyanate ayant une fonctionnalité au moins égale à 2, optionnellement, un co-polyol et des additifs.

Par « co-polyol », on entend un composé portant deux fonctions hydroxyles (type diol) ou plus (polyol) ajouté à la composition comprenant le polyol polyester pour en ajuster les propriétés telle que la fonctionnalité ou la viscosité, pour créer des nœuds de réticulation ou être allongeur de chaîne. Les co-polyols peuvent être en C2 à C8, préférentiellement en C3 à C7, avantageusement en C4 à C6. Les copolyols peuvent être avantageusement choisis parmi l'éthylène glycol, le glycérol, le sorbitol, l'érythritol, le xylitol, l'arabitol, le ribitol, le dulcitol, le mannitol et le volémitol. Les co-polyols préférés sont le glycérol et le sorbitol.

Typiquement, le(s) co-polyol(s) est ajouté dans un ratio polyol polyester/co-polyol(s) de 70/30, à 99/1, préférentiellement, 75/25 à 95/5, encore plus préférentiellement, 80/20 et 92/8, typiquement, 82/8 et 90/10, par exemple 85/15.

Selon l'invention, la composition comprend deux co-polyols typiquement un co-polyol en C2 et un co-polyol en C3 ou un co-polyol en C2 et un co-polyol en C5 ou un co-polyol en C2 et un co-polyol en C6 ou un co-polyol en C3 et un co-polyol en C5 ou un co-polyol en C3 et un co-polyol en C6 ou un co-polyol en C5 et un co-polyol en C6, ou deux co-polyols en C3 ou deux co-polyols en C5 ou deux co-polyols en C6.

Avantageusement, la composition comprend au moins un co-polyol en C2, typiquement, deux co-polyols, par exemple un co-polyol en C2 et un co-polyol en C5 ou C6, typiquement, l'éthylène glycol et le glycérol, l'éthylène glycol et l'érythritol, l'éthylène glycol et le xylitol, l'éthylène glycol et l'arabitol, l'éthylène glycol et le ribitol, l'éthylène glycol et le dulcitol, l'éthylène glycol et le mannitol ou l'éthylène glycol et le volémitol. Selon l'invention, le mélange de co-polyols préféré est le glycérol et l'éthylène glycol.

Avantageusement, la composition comprend au moins un co-polyol en C3, typiquement, deux co-polyols, par exemple, un co-polyol en C3 et un co-polyol en C5 ou C6, typiquement, le glycérol et le sorbitol, le glycérol et l'érythritol, le glycérol et le xylitol, le glycérol et l'arabitol, le glycérol et le ribitol, le glycérol et le dulcitol, le glycérol et le mannitol ou le glycérol et le volémitol. Selon l'invention, le mélange de co-polyols préféré est le glycérol et le sorbitol.

Avantageusement, la composition comprend au moins un co-polyol en C5 ou en C6, typiquement, deux co-polyols, par exemple, un co-polyol en C5 et un co-polyol en C6 ou deux co-polyols en C5 ou en C6.

Par exemple, la composition comprend deux co-polyols typiquement, l'érythritol et le sorbitol, le xylitol et le sorbitol, l'arabitol et le sorbitol, le ribitol et le sorbitol, le dulcitol et le sorbitol, le mannitol et le sorbitol ou le volémitol et le sorbitol.

Avantageusement, la composition comprend deux co-polyols typiquement dans un ratio C3/C6 ou C3/C5 ou C5/C6 compris entre 95/05 à 50/50, préférentiellement, 90/10 à 55/45, préférentiellement 87/13 à 60/40, plus préférentiellement 85/15 à 62/38, encore plus préférentiellement 80/20 à 65/35. Selon l'invention, le ratio préféré est 66/33, ratio particulièrement avantageux dans le cadre du mélange de co-polyols glycérol/sorbitol, notamment pour un mélange

final polyol polyester/glycérol/sorbitol de 85/10/5. Par « polyisocyanate », on entend tout composé chimique comprenant au moins deux fonctions chimiques isocyanate (NCO) distinctes, autrement dit, ayant « une fonctionnalité au moins égale à 2 ». Lorsque le polyisocyanate à une fonctionnalité de 2, on parle de di-isocyanate. Par fonctionnalité, on entend, au sens de la présente invention, le nombre total de fonctions isocyanate réactives par molécule d'isocyanate. La fonctionnalité d'un produit est évaluée par la titration de la fonction NCO par une méthode de dosage en retour de la dibultylamine excédentaire par l'acide chloridrique. Typiquement, ledit polyisocyanate a une fonctionnalité entre 2 et 5, typiquement 2,2 et 4, préférentiellement entre 2,5 et 3,5 encore plus préférentiellement entre 2,7 et 3,3. Avantageusement, ledit polyisocyanate est choisi parmi des polyisocyanates arômatiques, aliphatiques, cycloaliphatiques et leurs mélanges. On peut citer par exemple le 2,4-toluène diisocyanate, le 2,6-toluène diisocyanate, les mélanges de 2,4-et 2,6-toluène diisocyanate, le m-phénylène diisocyanate, le p-phénylène diisocyanate, le cis / trans de cyclohexane diisocyanate hexaméthylène diisocyanate, le m- et le p-tétraméthylxylylène-diisocyanate, m-xylylène, p-xylylène diisocyanate, le naphtalène-m, m-diisocyanate, le 1,3,5-hexaméthyl mésitylène triisocyanate, 1-méthoxyphényl-2,4-diisocyanate, 4,4'-diphényl-méthane diisocyanate, 4,4'-diisocyanabiphénylène 3,3'-diméthoxy-4,4'-diphényl diisocyanate, 3,3'-dimethyl-4,4'-diphényl diisocyanate, 4,4",4"-triphénylméthane triisocyanate, le toluène-2,4,6m-triisooyanate, 4,4'-diméthyl diphényl méthane-2,2', 5,5'-tétraisocyanate, et les isocyanates aliphatiques, tels que le 4,4'-diphénylméthane diisocyanate hydrogéné, toluène diisocyanate (TDI) hydrogénée et hydrogenatem méta- et paraxylène diisocyanate de tétraméthylxylylène diisooyanate (TMXDI®isooyanate, produit de American Cyanamid co, Wayne, NJ, USA.), 3: 1 méta-tétraméthylxylylène diisocyanate / triméthylolpropane (Cythane 3160® isocyanate, de la société American Cyanamid Co.), les molécules plurifonctionnelles tel que le poly-diisocyanate de diphénylméthylène (pMDI) et leurs analogues. Typiquement le polyisocyanate est choisi parmi le toluène diisocyanate (TDI), 4,4'-diphénylméthane diisocyanate (ou 4,4'-diisocyanate de diphénylméthylène ou 4,4'-MDI), le polyméthylène polyphénylène polyisocyanate (MDI polymérique, pMDI) et leur mélange.

Par « catalyseur de réaction », on entend un composé qui introduit en faible quantité accélère la cinétique de la formation de la liaison uréthane (-NH-CO-O-) par réaction entre le polyol polyester de l'invention et un polyisocyanate ou active la réaction entre un polyisocyanate et l'eau ou active la trimérisation des isocyanates. Typiquement les catalyseurs de réaction sont choisis parmi des amines tertiaires (tel que le diméthylcyclohexane), des dérivés d'étain (tel que le dibutyldilaurate d'étain), sels d'ammonium (tels que le méthanaminium N,N,N-trimethyl de 2,2-dimethylpropanoate) des carboxylates de métaux alcalins (tel que le 2-éthylhexanoate de potassium ou l'octoate de potassium) les ethers d'amine (tel que le bis(2-dimethylaminoéthyle) ether), et les triazines (tel que le 1,3,5-Tris(3-(dimethylamino)propyl))hexahydro-1,3,5-triazine).

Avantageusement, une composition destinée à l'obtention d'une mousse comprend ledit polyol polyester selon l'invention ou ledit polymère selon l'invention notamment le pré-polymère, un catalyseur de réaction, un polyisocyanate ayant une fonctionnalité au moins égale à 2, un agent gonflant et optionnellement, un stabilisant, un ignifugeant.

Avantageusement, lorsque la composition est une mousse ou une composition permettant l'obtention d'une mousse, le polyol polyester préféré est un polyol polyester à pH neutre et/ou comprend un sorbitol au titre de sucre alcool Z. Typiquement, le polyol polyester préféré est le bis(1,2-éthanediol)-sorbitol-diadipate, le bis(1,6-hexanediol)-sorbitol-diadipate ou le bis(1,4-butanediol)-sorbitol-diadipate, plus préférentiellement, le bis(1,4-butanediol)-sorbitol-diadipate, ou le bis(1,6-hexanediol)-sorbitol-diadipate.

Selon l'invention, une mousse comprend après polymérisation typiquement, un polymère selon l'invention notamment un polymère réticulé, un catalyseur de réaction, un agent gonflant et optionnellement, un stabilisant, au moins un co-polyol.

Typiquement, le terme « mousse » tel qu'utilisé par exemple dans les expressions « mousse de polyuréthane » ou « mousse polyisocyanurate », on entend un composé à structure alvéolaire tridimensionnelle de type expansé. Ladite mousse peut être rigide ou souple, à cellules ouvertes ou fermées. On parle de polyuréthane rigide (PUR), ou de polyisocyanurate rigide (PIR) pour des mousses rigides de polyuréthane ou de polyisocyanurate.

Par « mousse à cellules fermées » on entend une mousse dont la structure alvéolaire comporte des parois entre chaque alvéole constituant un ensemble de cellules jointes et distinctes permettant l'emprisonnement d'un gaz d'expansion. Une mousse est qualifiée de mousse à cellules fermées lorsqu'elle présente un maximum de 10% de cellules ouvertes. Typiquement les mousses à cellules fermées sont majoritairement des mousses rigides.

Par « mousse à cellules ouvertes » on entend une mousse dont la structure alvéolaire est constituée d'une matrice alvéolaire continue à parois ouverte entre les cellules ne permettant pas l'emprisonnement d'un gaz d'expansion. Une telle mousse permet la création de chemins de percolation au sein de sa matrice alvéolaire. Typiquement, les mousses à cellules ouvertes sont majoritairement des mousses souples.

Par « agent gonflant » on entend un composé induisant par une action chimique et/ou physique une expansion d'une composition au cours d'une étape de moussage. Typiquement, l'agent gonflant chimique est choisi parmi l'eau, l'acide formique, l'anhydride phtalique et l'acide acétique. L'agent gonflant physique est choisi parmi le pentane et les isomères du pentane, les hydrocarbures, les hydrofluorocarbures, les hydrochlorofluorooléfines, les hydrofluoro-oléfines (HFOs), les éthers et leurs mélanges. On peut citer le méthylal au titre d'exemple d'un agent gonflant de type éther.

Selon l'invention, un mélange d'agent gonflant chimique et physique préféré est par exemple un mélange eau/isomère du pentane ou acide formique/isomère du pentane ou eau/hydrofluoro-oléfines ou isomère de pentane / méthylal/eau ou encore eau/méthylal.

Par « stabilisant » on entend un agent permettant la formation d'une émulsion entre le polyol et l'agent gonflant ainsi que la stabilité physique de la matrice polymère lors de l'avancement des réactions notamment par la nucléation des gouttelettes d'agent gonflant, la stabilisation anti-coalescente pendant la polymérisation. Typiquement, les stabilisants sont choisis parmi l'un quelconque des copolymères silicone glycol (par exemple Dabco DC198 ou DC193 commercialisé par Air Products), copolymère non hydrolysable silicone glycol (par exemple DC5000from Air Products), copolymère de polyalkylène siloxane (par exemple Niax L-6164 de Momentive), copolymère méthylsiloxane polyoxyalkylène (par exemple Niax L-5348 de Momentive), copolymère polyetherpolysiloxane (par exemple Tegostab B8870 ou Tegostab B1048 de Evonik), copolymère de polydiméthylsiloxane polyéther (par exemple Tegostab B8526 de Evonik), polyéthersiloxane (par exemple Tegostab B8951 de Evonik), un copolymère polyéther-polysiloxane modifié (par exemple Tegostab B8871 de Evonik), un copolymère de polysiloxane polyoxyalkylene à block (par exemple de Tegostab BF 2370 de Evonik) et leurs dérivés ou leurs mélanges.

Par « additifs », on entend des agents tels que des anti-oxydants (agents de neutralisation des extrémités de chaînes à l'origine de la dépolymérisation ou chaînes co-monomères capables d'arrêter la propagation de dépolymérisation), des agents démoulant (talc, solution paraffine, silicone), des anti-hydrolyses, des biocides, des agents anti-UV (l'oxyde de titane, le triazine, le benzotriazole) et/ou des ignifugeants (antimoine, composés phosphorées, borées, azotées).

Par « agent ignifugeant », on entend un composé ayant la propriété de réduire ou d'empêcher la combustion ou l'échauffement des matériaux qu'elle imprègne ou recouvre, on parle de retardant de flamme ou de feu. On peut citer par exemple seul ou en mélange, le graphite, les silicates, le bore, les dérivés halogénés ou phosphorés tels que le Tris (1-chloro-2-propyl) phosphate (TCPP), le triéthylène de phosphate (TEP), les esters phosphate de triaryle, le polyphosphate d'ammonium, le phosphore rouge, le trishalogénaryle, et leur mélange.

Un exemple de composition selon l'invention permettant l'obtention d'une mousse rigide à cellules fermées est typiquement formulée avec un index de 115 soit un rapport NCO/OH de 1,15. Typiquement, une telle composition comprend au moins 50 à 100 parts d'un polyol polyester selon l'invention, 0 à 50 parts d'un co-polyol, 150 à 500 parts d'un polyisocyanate, 0,5 à 5 parts d'un catalyseur typiquement d'un catalyseur aminé tel que la diméthylcyclohexyleamine, 0,5 à 6 parts d'un agent gonflant tel que l'eau, 0 à 5 parts d'un stabilisant tel qu'un copolymère polyéther-polysiloxane et 0 à 20 parts d'un agent ignifugeant.

Une mousse de polyuréthane rigide à cellules fermées comprend par exemple 100 parts d'un polyol polyester, 270 parts d'un polyisocyanate, 2 parts d'un catalyseur aminé tel que la diméthylcyclohexyleamine, 6 parts d'un agent gonflant tel que l'eau, 2,5 parts d'un stabilisant tel qu'un copolymère polyéther-polysiloxane et 10 parts d'un agent ignifugeant.

Un exemple d'une composition permettant l'obtention d'une mousse rigide polyisocyanurate à cellules fermées est typiquement formulée avec un index minimum de 200 ou un minimum de 300 soit un ratio NCO /OH supérieur à 2.0 ou supérieur à 3, préférentiellement un index compris entre 250 et 450, encore plus préférentiellement compris entre 300 et 400 soit un ratio NCO/OH préférentiellement compris entre 2,5 et 4,5, encore plus préférentiellement compris entre 3,0 et 4,0. Une composition permettant l'obtention d'une mousse rigide polyisocyanurate à cellules fermées comprend, par exemple 50 à 100 parts du polyol polyester selon l'invention, 0 à 50 parts d'un co-polyol, 150 à 500 parts d'un polyisocyanate, 0,1 à 13 parts d'au moins un catalyseur, préférentiellement deux catalyseurs, typiquement un catalyseur aminé et un carboxylate de potassium (par exemple dans un ratio catalyseur aminé / carboxylate de potassium de 0,5 à 2), 0 à 20 parts d'un agent gonflant tel qu'un isomère du pentane, 0 à 8 parts d'un stabilisant et 0 à 20 parts d'un agent ignifugeant.

Typiquement, une composition permettant l'obtention d'une mousse rigide polyisocyanurate à cellules fermées comprend par exemple, 100 parts du polyol polyester selon l'invention ; 173 parts d'un polyisocyanate tel que le polydiisocyanate de diphénylméthylène ; 0,5 parts d'un catalyseur aminé tel que la dimethylcyclohexane ; 2,5 parts d'un carboxylate de potassium tel que le 2-ethylehexanoate de potassium ; 18 parts d'un agent gonflant tel qu'un isomère du pentane ; 2,5 parts d'un stabilisant et 10 parts d'un agent ignifugeant.

Les mousses de polyuréthane ou de polyisocyanurate rigides sont par exemple utilisées comme panneau rigide pour l'isolation thermique typiquement, des bâtiments.

Une composition permettant l'obtention d'une mousse de polyuréthanne souple présente typiquement un index de 100 à 120 et comprend par exemple 40 à 60% en masse d'un polyol polyester selon l'invention, 1 à 10% en masse d'agent gonflant typiquement de l'eau, 40 à 60% en masse de polyisocyanate tel que le diisocyanate de toluène, 0 à 5% en masse d'un stabilisant, 0,1 à 5% en masse d'au moins un catalyseur.

Une composition de mousse de polyuréthanne souple comprend par exemple 100 parts du polyol polyester selon l'invention ; 5 parts d'agent gonflant tel que l'eau ; 50 parts de polyisocyanate tel que le diisocyanate de toluène 1 part de stabilisant tel que le Tegostab BF 2370 ; 0,2 parts de catalyseur tel que le dibutyldilaurate d'étain.

Typiquement, les mousses de polyuréthane souples sont utilisées pour les matelas, les sièges automobiles, le mobilier, l'isolation phonique et l'acoustique.

Par « élastomère » tel que dans l'expression « élastomère de polyuréthane » on entend un matériau présentant des propriétés élastiques pouvant subir de grandes déformations et revenir à son état initial sans perte de propriétés. Une composition permettant l'obtention d'un d'élastomère de polyuréthane comprend typiquement, un polyol polyester selon l'invention ou un polymère selon l'invention typiquement, un prépolymère selon l'invention, un polyisocyanate et au moins un co-polyol, préférentiellement un diol.

Typiquement, une composition permettant l'obtention d'un élastomère de polyuréthane présente un ratio NCO/OH compris entre 0,1 et 3 ; préférentiellement entre 0,5 et 2.

Avantageusement, une composition permettant l'obtention d'un élastomère comprend 3 à 15% en masse de polyisocyanate tel que le 4,4'-diphénylméthane diisocyanate (4,4'-MDI), 25 à 75% en masse d'un polymère de polyol polyester selon l'invention de masse molaire préférentiellement comprise entre 2000 et 6500 g/mol ; 10 à 30% en masse d'un co-polyol typiquement, un diol tel qu'un diol en C3 à C8. Avantageusement, ledit polyol polyester forme les segments souples de l'élastomère, et le diol associé au polyisocyanate les segments rigides de l'élastomère.

Par « revêtement » tel que dans l'expression « revêtement de polyuréthane », on entend un matériau destiné à recouvrir une surface en vue de sa protection, il peut être par exemple fin et résistant ou épais afin d'absorber les chocs.

Typiquement, une composition permettant l'obtention d'un revêtement de polyuréthane présente un ratio NCO/OH supérieur à 1, préférentiellement compris entre 1,1 et 3.

Un exemple de composition permettant l'obtention d'un revêtement de polyuréthane comprend :

- 10 à 60% en masse d'un polymère selon l'invention typiquement, un prépolymère issu de la réaction entre un polyol polyester de l'invention préférentiellement de masse molaire 2000 g/mol et un polyisocyanate tel que le 4,4'-MDI ; le ratio NCO/OH polymère selon l'invention étant >1 typiquement le ratio étant compris entre 1,1 et 3,
- 0 à 5% en masse de pigments ou de pâte pigmentaire, tels que par exemple les pigments azoïques,
- 0 à 2% en masse d'un catalyseur par exemple la dibutyltin dilaurate (DBTDL)
- optionnellement, 0 à 5% d'additifs tels que par exemple :
    - ◦ 0 à 5% en masse d'additifs rhéologiques tels que par exemple des solvants ou diluants réactifs, ou des épaississants ;
    - ◦ 0 à 5% en masse d'additifs fonctionnels tels que des agents anti mousse, des agents anti-fongique ou leur mélange.

Par « additifs rhéologiques », on entend des additifs ayant pour fonction d'augmenter ou de réduire la viscosité de la composition, il s'agit de solvants, de diluants ou d'épaississant, on peut citer par exemple l'éthylène glycol.

Par « additifs fonctionnels », on entend des composés conférant à la composition une fonctionnalité particulière tels que des agents anti-mousse et notamment les polyéthers polysiloxanes tel que le Tergitol L (Dow), des agents anti-fongique tels que par exemple le pentachlorophénol.

Avantageusement, la polymérisation de la composition comprenant le pré-polymère selon l'invention débute après l'application de ladite composition de revêtement sur un support afin d'obtenir un revêtement réticulé. Typiquement, la réaction de polymérisation s'effectue avec l'humidité de l'air.

Par « adhésif » ou « composition adhésive » on entend une composition permettant l'adhésion entre deux composés, surfaces ou objets, un exemple de composition adhésive comprend un polyol polyester de l'invention, un polyisocyanate avec un ratio NCO/OH compris entre 1 et 1,6 et un catalyseur tel que le dibutyldilaurate d'étain typiquement, à 0,05 % w/w.

L'invention concerne également un procédé d'obtention d'une mousse, d'un revêtement, d'un adhésif ou d'un élastomère de polyuréthane ou de polyisocyanurate comprenant une étape d'obtention d'un polyol polyester selon l'invention ou d'un prépolymère selon l'invention, une étape d'addition d'au moins un polyisocyanate, d'au moins un agent gonflant, d'un stabilisant et d'au moins un catalyseur de réaction, et une étape de polymérisation et optionnellement un ignifugeant.

L'invention concerne enfin une utilisation d'un polyol polyester selon l'invention avantageusement choisi parmi le bis(1,2-éthanediol)-sorbitol diadipate, le bis(1,3-propanediol)-sorbitol diadipate, le bis(1,4-butanediol) -sorbitol diadipate, le bis(1,6-hexanediol)-sorbitol diadipate, le bis(1,8-octanediol)-sorbitol diadipate, le bis(1,10-décanediol-)sorbitol diadipate, le bis(1,12-dodécanediol)-sorbitol diadipate, le bis(1,4-butanediol)-sorbitol disuccinate, ou le sorbitol- diadipate- sorbitol dans la fabrication d'une mousse, d'un revêtement, d'un adhésif ou d'un élastomère de polyuréthane ou de polyisocyanurate.

Bien qu'ayant des significations distinctes, les termes « comprenant », « contenant », « comportant » et « consistant en » ont été utilisés de manière interchangeable dans la description de l'invention, et peuvent être remplacés l'un par l'autre. L'invention sera mieux comprise à la lecture des figures et exemples suivants donnés uniquement à titre

d'exemple.

## FIGURES

**La figure 1** correspond aux spectres de RMN $^{13}C$ de l'intermédiaire réactionnel bi-acidique commun à l'ensemble des polyols polyesters où Y=Y'=acide adipique **(fig. 1a)**, spectre de RMN $^{13}C$ du bis(1,2 éthanediol)-sorbitol diadipate **(fig. 1b)**, spectre de RMN $^{13}C$ bis(1,3-propanediol)-sorbitol diadipate (**fig. 1c**), spectre de RMN $^{13}C$ bis(1,4-butanediol)-sorbitol diadipate (**fig. 1d**)

**La figure 2** correspond aux spectres de RMN $^{13}C$ bis(1,6 hexanediol)-sorbitol diadipate (**fig. 2a**), spectre de RMN $^{13}C$ bis(1,8-octanediol)-sorbitol diadipate (**fig. 2b**), spectre de RMN $^{13}C$ bis(1,10-décanediol)-sorbitol diadipate (**fig. 2c**), spectre de RMN $^{13}C$ bis(1,12-dodécanediol)-sorbitol diadipate (**fig. 2d**).

## EXEMPLES

### Exemple 1

1.1 Matériels et méthodes :

Le D-sorbitol commercialisé par TEREOS SYRAL (sorbitol sup. 98%, eau inf. 0,5%, sucres réducteurs inf. 0,1%) ; le 1,4 butanediol (99%), le 1,10 décanediol, le 1,2-éthanediol, le 1,6-hexanediol, le 1,10-octanediol, le 1,12-dodecanediol sont commercialisés par la société SIGMA ALDRICH ; le 1,8 octanediol (98%) et le Glycérol sont commercialisés par la société FLUKA ; l'acide adipique (99%) commercialisé par la société ACROS ORGANICS ; le 1,3-propanediol par ALFA AESAR et l'acide succinique (Technical grade) commercialisé par la société BIOAMBERT.

Le sorbitol et l'acide adipique sont séchés dans une étuve à pression réduite à 45°C pendant une nuit puis sont stockés en dessiccateur sous vide. Les autres produits sont utilisés tels quels.

*Synthèse de bis(1,2-éthanediol)-sorbitol diadipate (bis(2-hydroxylethyl) O,O'-((2R, 3R,4R, 5S)-2,3,4,5-tetrahydroxyhexane- 1, 6-diyl) diadipate)*

Dans un ballon tricol (trois cols de rodage 29 :32) de 250mL, 10,00g de sorbitol (0.0549mol) et 16.03g d'acide adipique (0,11mol) sont introduits. L'entrée d'argon est fixée sur le premier col du ballon tricol, sur le second (col central) est fixé le système complet de distillation à colonne courte. Enfin le troisième et dernier col est bouché à l'aide d'un bouchon de type septum perforable pour permettre de futures injections dans le milieu réactionnel. Le milieu réactionnel est porté à 150°C sous atmosphère inerte (argon) et sous une faible agitation réglée à 100 rotation par minute (rpm) qui sera augmentée à 350rpm une fois que le milieu réactionnel est totalement visqueux. Après trois heures de réaction, 6.83g de 1,2-éthanediol (0,11mol) sont injectés à l'aide d'une seringue de 12mL puis la réaction est poursuivie pendant 6h pour un temps total de réaction de 9h. A 8h00 et 9h00 de réaction deux passages sous vide de respectivement 30 secondes et 1 minute sont effectués pour tirer l'eau produite par la réaction de polycondensation. Après chaque passage sous vide, le milieu réactionnel est ramené sous atmosphère inerte et à pression atmosphérique.

*Synthèse du bis(1,3-propanediol)-sorbitol diadipate (bis(3-hydroxypropyl) O,O'-((2R, 3R,4R, 5S)-2,3,4,5-tetrahydroxyhexane- 1, 6-diyl) diadipate)*

Dans un ballon tricol de 250mL 2,00g de sorbitol (0,0110mol), 3.2g d'acide adipique (0,0219mol) et un agitateur magnétique sont introduits. Le montage du ballon tricol et du système de distillation est effectué comme précédemment. Le milieu réactionnel est porté à 150°C sous atmosphère inerte (argon) et sous une faible agitation réglée à 100rpm qui sera augmentée à 350rpm une fois que le milieu réactionnel est totalement visqueux. Après trois heures de réaction, 1.67g de 1,3-propanediol (0.0219mol) sont injectés à l'aide d'une seringue de 12mL puis la réaction est poursuivie pendant 6h soit un temps total de réaction de 9h. A 3h30 et 6h30 de réaction deux passages sous vide de respectivement 30 secondes et 1 minute sont effectués pour tirer l'eau produite par la réaction de polycondensation. Après chaque passage sous vide, le milieu réactionnel est ramené sous atmosphère inerte et à pression atmosphérique.

*Synthèse du bis(1,4-butanediol)-sorbitol diadipate(bis(4-hydroxybutyl)O,O'-((2R, 3R,4R, 5S)-2,3,4,5-tetrahydroxyhexane- 1, 6-diyl) diadipate)*

Dans un ballon tricol de 250mL, 10,00g de sorbitol (0,0549mol) et 16,03g d'acide adipique (0.11mol) ainsi

qu'un agitateur magnétique sont introduits. Le montage du ballon tricol et du système de distillation est effectué comme précédemment. Le milieu réactionnel est porté à 150°C sous atmosphère inerte (argon) et sous une faible agitation réglée à 100rpm qui sera augmentée à 350rpm une fois que le milieu réactionnel est totalement visqueux. Après trois heures de réaction, 9,98g de 1,4-butanediol (0,11mol) sont injectés à l'aide d'une seringue de 12mL puis la réaction est poursuivie pendant 6h soit un temps total de réaction de 9h. A 3h30 et 6h30 de réaction deux passages sous vide de respectivement 30 secondes et 1 minute sont effectués pour tirer l'eau produite par la réaction de polycondensation. Après chaque passage sous vide, le milieu réactionnel est ramené sous atmosphère inerte et à pression atmosphérique.

*Synthèse du bis(1,6-hexanediol)-sorbitol diadipate (bis(6-hydroxyhexyl)O,O'-((2R, 3R,4R, 5S)-2,3,4,5-tetrahydroxyhexane- 1, 6-diyl) diadipate)*

Dans un ballon tricol de 250mL, 2,00g de sorbitol (0,0110mol) et 3,2g d'acide adipique (0,0219mol) ainsi qu'un agitateur magnétique sont introduits. Le montage du ballon tricol et du système de distillation est effectué comme précédemment. Le milieu réactionnel est porté à 150°C sous atmosphère inerte (argon) et sous une faible agitation réglée à 100rpm qui sera augmentée à 350rpm une fois que le milieu réactionnel est totalement visqueux. Après trois heures de réaction, 9,98g de 1,6-hexanediol (0,0219mol) sont injectés à l'aide d'une seringue de 12mL puis la réaction est poursuivie pendant 6h soit un temps total de réaction de 9h. A 3h30 et 6h30 de réaction deux passages sous vide de respectivement 30 secondes et 1 minute sont effectués pour tirer l'eau produite par la réaction de polycondensation. Après chaque passage sous vide, le milieu réactionnel est ramené sous atmosphère inerte et à pression atmosphérique.

*Synthèse du bis(1,8-octanediol)-sorbitol diadipate (bis(8-hydroxyoctyl) O,O'-((2R, 3R,4R, 5S)-2,3,4,5-tetrahydroxyhexane- 1, 6-diyl) diadipate)*

Dans un ballon tricol de 250mL sont introduits 1,000g de sorbitol (0,00549mol), 1,603g d'acide adipique (0,01098mol) ainsi qu'un agitateur magnétique. Le montage du ballon tricol et du système de distillation est effectué comme précédemment. Le milieu réactionnel est porté à 150°C sous atmosphère inerte (argon) et sous une faible agitation à 100rpm puis à 350rpm une fois que le milieu réactionnel est totalement visqueux. Après trois heures de réaction, 1.605g de 1,8-octanediol (0,01098mol) sont injectés puis la réaction est poursuivie pendant 6h soit un temps total de réaction de 9h. A 3h30 et 6h30 de réaction deux passages sous vide de respectivement 30 secondes et 1 minute sont effectués pour tirer l'eau produite par la réaction de polycondensation. Après chaque passage sous vide, le milieu réactionnel est ramené sous atmosphère inerte et à pression atmosphérique.

*Synthèse du* bis(1,10-décanediol)-sorbitol *diadipate (bis(10-hydroxydecyl) O,O'-((2R, 3R,4R, 5S)-2,3,4,5-tetrahydroxyhexane- 1, 6-diyl) diadipate)*

Dans un ballon tricol de 250mL, 1,000g de sorbitol (0,00549mol) et 1,603g d'acide adipique (0,01098mol) ainsi qu'un agitateur magnétique sont introduits. Le montage du ballon tricol et du système de distillation est effectué comme précédemment. Le milieu réactionnel est porté à 150°C sous atmosphère inerte (argon) et sous une faible agitation à 100rpm puis à 350rpm une fois que le milieu réactionnel est totalement visqueux. Après trois heures de réaction, 1,910g de 1,10-octanediol (0,01098mol) sont injectés puis la réaction est poursuivie pendant 6h soit un temps total de réaction de 9h. A 3h30 et 6h30 de réaction deux passages sous vide de respectivement 30 secondes et 1 minute sont effectués pour tirer l'eau produite par la réaction de polycondensation. Après chaque passage sous vide, le milieu réactionnel est ramené sous atmosphère inerte et à pression atmosphérique.

*Synthèse du bis(1,12-dodécanediol)-sorbitol diadipate (bis(12-hydroxydodecyl) O,O'-((2R, 3R,4R, 5S)-2,3,4,5-tetrahydroxyhexane- 1,6-diyl) diadipate)*

Dans un ballon tricol de 250mL, 5,00g de sorbitol (0,0274mol) et 8,015g d'acide adipique (0,0549mol) ainsi qu'un agitateur magnétique sont introduits. Le montage du ballon tricol et du système de distillation est effectué comme précédemment. Le milieu réactionnel est porté à 150°C sous atmosphère inerte (argon) et sous une faible agitation réglée à 100rpm qui sera augmenté à 350rpm une fois que le milieu réactionnel est totalement visqueux. Après trois heures de réaction, 11,089g de 1,12-dodecanediol (0,0549mol) sont injectés à l'aide d'une seringue de 12mL puis la réaction est poursuivie pendant 6h soit un temps total de réaction de 9h. A 3h30 et 6h30 de réaction deux passages sous vide de respectivement 30 secondes et 1 minute sont effectués pour tirer l'eau produite par la réaction de polycondensation. Après chaque passage sous vide, le milieu réactionnel est ramené sous atmosphère inerte et à pression atmosphérique.

*Synthèse du bis(1,4-butanediol)-sorbitol disuccinate (bis(4-hydroxybutyl) O,O'-((2R, 3R, 4R, 5S)-2,3,4,5-tetrahydroxyhexane-1, 6-diyl) disuccinate)*

Dans un ballon tricol de 250mL, 10,00g de sorbitol (0,0549mol) et 12.95g d'acide succinique (0,11mol) ainsi qu'un agitateur magnétique sont introduits. Le montage du ballon tricol et du système de distillation est effectué comme précédemment. Le milieu réactionnel est porté à 150°C sous atmosphère inerte (argon) et sous une faible agitation réglée à 100rpm qui sera augmenté à 350rpm une fois que le milieu réactionnel est totalement visqueux. Après trois heures de réaction, 9,98g de 1,4-butanediol (0,11mol) sont injectés à l'aide d'une seringue de 12mL puis la réaction est poursuivie pendant 6h soit un temps total de réaction de 9h. A 3h30 et 6h30 de réaction deux passages sous vide de respectivement 30 secondes et 1 minute sont effectués pour tirer l'eau produite par la réaction de polycondensation. Après chaque passage sous vide, le milieu réactionnel est ramené sous atmosphère inerte et à pression atmosphérique.

*Synthèse du sorbitol-diadipate-disorbitol (2S,3R,4R,5R)-2,3,4,5,6-pentahydroxyhexyl ((2R, 3R, 4R, 5S)-2, 3, 4, 5-tetrahydroxy-6-((6-oxo-6-(((2R, 3R, 4R, 5S)-2,* 3, 4, *5,6-pentahydroxyhexyl)oxy)hexanoyl)oxy)hexyl) adipate*

Dans un ballon tricol de 250mL, 10,00g de sorbitol (0,0549mol) et 16,03g d'acide adipique (0,11mol) ainsi qu'un agitateur magnétique sont introduits. Le montage du ballon tricol et du système de distillation est effectué comme précédemment. Le milieu réactionnel est porté à 150°C sous atmosphère inerte (argon) et sous une faible agitation réglée à 100rpm qui sera augmentée à 350rpm une fois que le milieu réactionnel est totalement visqueux. Après trois heures de réaction, 20,0g de sorbitol (0,11mol) sont injectés à l'aide d'une seringue de 12mL puis la réaction est poursuivie pendant 6h soit un temps total de réaction de 9h. A 3h30 et 6h30 de réaction deux passages sous vide de respectivement 30 secondes et 1 minute sont effectués pour tirer l'eau produite par la réaction de polycondensation. Après chaque passage sous vide, le milieu réactionnel est ramené sous atmosphère inerte et à pression atmosphérique.

*Synthèse du bis(1,4-butanediol)-sorbitol diadipate (bis(4-hydroxybutyl)O,O'-((2R,3R,4R,5S)-2,3,4,5-tetrahydroxyhexane-1,6-diyl) diadipate) et 1,4-butanediol-sorbitol adipate modifié en réacteur:*

La réaction est effectuée dans un réacteur étanche en Inox équipé d'une pâle d'agitation en U, d'un Dean Stark présentant une sortie en haut du condensateur pour pouvoir y lier une pompe à vide et d'une sortie basse pour récupérer les condensats, d'une entrée et d'une sortie de gaz inerte. Dans le réacteur, sont introduit à l'état de poudre du sorbitol et de l'acide adipique dans un ratio molaire 1/2 (sorbitol/acide adipique). Le réacteur est mis sous atmosphère inerte puis est lancé en chauffe. Lorsque la température atteint 100°C, l'agitation est progressivement lancée jusqu'à 170rpm. Quand la température atteint 150°C, la réaction est lancée et poursuivie durant 3h. Au bout de 3h, du 1,4 butanediol (appelé diol par la suite) est introduit dans le réacteur dans un ratio molaire (1,4 butanediol/sorbitol) 2,2/1. Lorsque la température du milieu réactionnel revient à 150°C (agitation toujours maintenue à 170rpm, atmosphère inerte) le diol est rajouté après 2h30 puis un passage sous vide partiel est effectué pendant une durée d'une minute puis la pression atmosphérique est ramenée sous atmosphère inerte. 4h30 après l'ajout de diols, un nouveau flush de vide partiel est effectué pendant 2 minutes puis la pression atmosphérique est ramenée sous atmosphère inerte. 6h15minutes après l'introduction du diol (soit un temps total de réaction de 9h15min à 150°C), le réacteur est arrêté et le produit de réaction est récupéré à chaud afin d'avoir un minimum de perte lors du transfert de matière du réacteur vers le conditionnement du produit.

*1.2 Préparation des échantillons pour mesure de l'indice d'hydroxyle (IOH)*

La mesure de l'indice d'hydroxyle a été effectuée selon la norme ASTM 4274-99 dans laquelle la titration colorimétrique a été remplacée par une titration pH-métrique. Plus particulièrement, la mesure a été effectuée de la façon suivante. Dans un ballon monocol de 250mL, environ 1g d'échantillon sont pesés au 1mg près. 20mL d'une solution réactive d'anhydride phtalique 1N dans de la pyridine sont ajoutés à la pipette jaugée de 20mL puis le système est mis à reflux pendant 45min à 130°C. Après avoir refroidi le mélange, 10mL de pyridine sont introduits par le haut du réfrigérant puis le contenu du ballon est transvasé dans un bécher haut de 150mL pour titration. On ajoute ensuite 20mL de pyridine et 30mL d'eau avant de titrer par la potasse dans l'eau à 1N à l'aide du titrateur automatique.

*1.3 Préparation des échantillons pour analyse RMN $^{31}P$*

Environ 10-15mg de polyol sont introduits dans un flacon de 4mL auquel sont ajoutés 400$\mu$L de solvant (pyridine/$CDCl_3$ ratio 1,6/1, v/v), le mélange est laissé sous agitation magnétique durant 2min. Sont ensuite ajoutés 100$\mu$L d'une solution de standard (1mmol de N-hydroxylnaphhalimide dans 10mL de solvant et 50mg de Cr(III) acétyle acétonate) et 100$\mu$L de réactif phosphoré (2-chloro-1,2,3-dioxaphospholane). L'ensemble est laissé sous agitation magnétique

à température ambiante pendant 2h avant l'analyse $^{31}P$ RMN.

*1.4 Neutralisation des polyols à base de sorbitol*

La réaction de polycondensation durant la synthèse des différents polyols ne permettant pas d'atteindre des taux de conversion de 100% des traces résiduelles d'acide non réagi sont alors retrouvées dans le produit final. Afin de s'en affranchir, trois types de neutralisation ont été envisagées. Deux neutralisations classiques de type acido-basiques avec de la soude ou de la potasse pour neutraliser les résidus acides par simple réaction acido-basique et une neutralisation par l'hexan-1-ol soit une estérification par un mono-alcool.

a) Neutralisation par la soude (NaOH)

Dans un ballon monocol de 250mL sont introduit: 50,4 g de 1,4 butanediol-sorbitol adipate, 2,065g de NaOH correspondant à l'acidité résiduelle à neutraliser (déterminé par titration) ainsi que 60mL d'éthanol pour fluidifier le milieu. Le ballon est équipé d'un réfrigérant à boule et le milieu est chauffé à 60°C. Le temps de réaction est d'une heure. Le produit de réaction est ensuite distillé avec un pont de distillation pour extraire l'éthanol et retrouver le polyol initial neutralisé.

b) Neutralisation par la potasse (KOH)

Dans un ballon monocol de 250mL sont introduits : 49,9 g de 1,4 butanediol-sorbitol adipate, 2,89g de KOH correspondant à l'acidité résiduelle à neutraliser (déterminé par titration) ainsi que 60mL d'éthanol pour fluidifier le milieu. Le ballon est équipé d'un réfrigérant à boule et le milieu est chauffé à 60°C. Le temps de réaction est d'une heure. Le produit de réaction est ensuite distillé avec un pont de distillation pour extraire l'éthanol et retrouver le polyol initial neutralisé.

c) Neutralisation avec l'hexanol

Dans un ballon monocol de 250mL sont introduits: environ 23,3 g de 1,4 butanediol-sorbitol adipate, ainsi que 170 mL d'hexanol. Le ballon est équipé d'un pont de distillation permettant le reflux de l'hexanol et l'élimination de l'eau produite par la réaction puis le milieu est porté à reflux pendant une nuit. Le produit de réaction est ensuite distillé sous vide à 130°C avec un pont de distillation.

*1.5 Caractérisation des produits obtenus*

Les masses molaires et les indices de polydispersité ont été déterminés par chromatographies d'exclusion stérique dans le chloroforme en utilisant une chromatographie liquide Shimadzu. Les colonnes utilisées sont des PLGel Mixed-Cet PLGeL de 100A. un détecteur différentiel d'indice de réfraction est utilisé. Le chloroforme est utilisé comme éluant à un débit de 0.8ml/min. L'appareil est calibré avec des standards de polystyrènes linéaires avec des masse molaires allant de 162 à 1 650 000 g/mol.

Les calculs des masses moléculaires moyennes et des indices de polymolécularité en détection RI ou UV sont effectués suivant une courbe d'étalonnage avec des étalons de polystyrène ou de polyméthylméthacrylate.

Les analyses thermogravimétriques (ATG) ont été effectuées sur TGA Q5000 de TA Instrument et les analyses ont été effectuées à l'aide du logiciel Universal Analysis 2000. La rampe de chauffe est réglée à 10°C/min de 25°C à 600°C sous un flux constant d'air reconstitué ou d'hélium à 25mL/min.

Les analyses calorimétriques différentielles (DSC) sont effectuées à l'aide d'une DSC Q200 de TA Instrument. Les rampes de chauffe sont réglées à 10°C/min et les rampes de refroidissement à 5°C/min entre -85°C et 160°C sous un flux de $N_2$ réglé à 25mL/min. Les données sont analysées à l'aide du logiciel Universal Analysis 2000.

Les titrations par retour sont effectuées à l'aide d'un titrateur automatique TitroLine 7000 fourni par SI Analitics. La solution titrante utilisée est obtenue à partir de concentrés volumétriques (Fixanal) fournis par Fluka Analytical.

La résonnance magnétique du phosphore ($^{31}P$) a été effectuée sur un spectromètre Bruker advance 3 400MHz. La fenêtre spectrale est centrée sur 22677Hz et est large de 100ppm (particules par millions). Le temps de relaxation est fixé à 2 secondes et le nombre de scans enregistré est de 128.

La résonnance magnétique du proton a été effectuée sur le même spectromètre avec un nombre de scans de 128.

La spectroscopie infrarouge a été effectuée avec un spectromètre infrarouge Nicolet 380 à transformation de Fourier (Thermo Electron Corporation). L'appareil est équipé d'un module à analyse de la réflexion totale atténuée (ATR) sur diamant avec une résolution de 4cm$^{-1}$, 64 scans d'analyse par échantillon avec une correction ATR propre au diamant

sont effectués.

Les mesures rhéologiques ont été effectuées sur un rhéomètre Anton Paar Physica MCR 301 avec une géométrie cône plan de diamètre 25mm et une enceinte à effet Peltier. Le programme d'analyse est constitué de trois étapes :

- 1 : une rampe en température de 0°C à 50°C à 0,1°C/min et un taux de cisaillement constant de 10 $s^{-1}$
- 2 : un balayage en taux de cisaillement allant de 0,001 $s^{-1}$ à 100 $s^{-1}$ à une température constante de 20°C
- 3 : un balayage en taux de cisaillement allant de 0.001 $s^{-1}$ à 100 $s^{-1}$ à une température constante de 25°C.

**Exemple 2 Résultats**

2.1 Analyse de la structure des produits obtenus et rendements

Les procédés de synthèse mis en œuvre ont permis l'obtention des 9 molécules suivantes :

- *bis(1,4-butanediol)-sorbitol* disuccinate
- sorbitol-diadipate-sorbitol
- *bis(1,2-éthanediol)-sorbitol* diadipate
- *bis(1,3-propanediol)-sorbitol* diadipate
- *bis(1,4-butanediol)-sorbitol* diadipate
- *bis(1,6-hexanediol)-sorbitol* diadipate
- bis(1,8-octanediol)-sorbitol diadipate
- bis(1,10-décanediol)-sorbitol diadipate
- bis(1,12-dodécanediol)-sorbitol diadipate

Le procédé selon l'invention permet d'obtenir les produits ci-dessus avec des rendements compris entre 80 et 90%. Les produits synthétisés ont des aspects macroscopiques différents par exemple :

- *bis(1,2-éthanediol)-sorbitol* diadipate : liquide visqueux légèrement jaune
- *bis(1,3-propanediol)-sorbitol* diadipate: liquide visqueux légèrement jaune
- *bis(1,4-butanediol)-sorbitol* diadipate : liquide visqueux légèrement jaune
- *bis(1,6-hexanediol)-sorbitol* diadipate : gel solide jaunâtre
- bis(1,8-octanediol)-sorbitol diadipate : cire orangée
- bis(1,10-décanediol)-sorbitol diadipate : cire blanchâtre
- bis(1,12-dodécanediol)-sorbitol diadipate : cire beige

*2.1.1 Analyse en spectroscopie infrarouge à transformée de Fourier (FT-IR)*

Le bis(1,2-éthanediol)-sorbitol diadipate, le bis(1,3-propanediol)-sorbitol diadipate, le bis(1,4-butanediol) -sorbitol diadipate, le bis(1,6-hexanediol)-sorbitol diadipate, le bis(1,8-octanediol)-sorbitol diadipate, le bis(1,10-décanediol)-sorbitol diadipate, le bis(1,12-dodécanediol)-sorbitol diadipate, ont été analysés par FT-IR. Pour chaque produit les bandes d'absorptions caractéristiques des fonctions hydroxyles (large bande à 3999 $cm^{-1}$), chaînes carbonés (bande d'absorption à 2940 $cm^{-1}$) et la bande correspondant aux fonctions ester (bande fine et intense à 1725 $cm^{-1}$) ont été observées. La présence de ces bandes d'absorption, plus particulièrement celles de la fonction ester confirme que la réaction d'estérification a bien eu lieu et que la structure des polyols est bien de type polyol polyester portant des fonctions hydroxyles nécessaires pour la formulation de mousse PUR et PIR.

Aucun co-produit de réaction ne ressort de l'analyse FT-IR, les fonctions acides résiduelles ne sont pas visibles en FT-IR car elles sont confondues dans le pied de la bande d'absorption caractéristique des fonctions esters. Comparativement aux procédés de l'art antérieur, le procédé de la présente invention n'induit pas ou très peu de cyclisation du sorbitol. En effet, aucune bande d'absorption à 1068$cm^{-1}$ caractéristique de la cyclisation du sorbitol n'est observée. Il est néanmoins possible que ces phénomènes de cyclisation soient présents de façon minoritaire et donc indétectables car inférieur à la limite de détection de la méthode utilisée, ou du fait de la superposition du signal concerné parmi d'autres bandes d'absorption dans la zone comprise entre 1500 et 940 $cm^{-1}$.

*2.1.2 Analyse des résultats par RMN du proton (RMN-$^1H$)*

L'analyse par résonnance magnétique du proton de l'ensemble des polyols synthétisés permet de suivre l'avancement de la réaction en suivant l'évolution du pic correspondant au proton en $\alpha$ de la liaison ester formée à 2,3 ppm et celui du proton en $\alpha$ de la fonction acide consommée à 2,2 ppm. Cela permet de déterminer les différents temps caractéristiques des réactions d'estérification mises en jeux. Lors de la première partie de la synthèse lorsque l'intensité relative du pic correspondant à la fonction acide a diminué de moitié, l'intermédiaire réactionnel recherché est obtenu avec un rendement de 100% et correspond au moment d'injection du diol terminal. En fin de réaction, le rapport des deux pics renseigne sur l'état d'avancement de la réaction entre l'intermédiaire réactionnel et le diol. L'avancement maximal atteint est de 85%.

*2.1.3 Analyse des résultats par RMN du carbone (RMN-$^{13}C$)*

L'analyse par résonnance magnétique du carbone permet d'assigner l'ensemble des atomes de carbone présents dans la molécule à un déplacement chimique. Cette analyse permet de déterminer le squelette carboné des différents polyols synthétisés. Les plages de déplacements chimiques servant de référence pour l'analyse des différents spectres sont les suivantes :

**Tableau 1 : Déplacements chimiques des différents carbones présents dans les polyols polyesters**

| Déplacements chimiques en ppm | Carbone correspondant |
|---|---|
| 23 à 26 | Chaînes carbonées non fonctionnalisées |
| 27 à 35 | Chaînes carbonées à proximité de fonctions ester ou alcool |
| 60 à 62 | Carbone portant une fonction hydroxyle primaire terminale |
| 62 à 64 | Carbone en $\alpha$ de l'ester sur la chaîne sorbitol |
| 64 à 75 | Chaîne carbonée du sorbitol (siqnal faible) |
| 172 à 173 | Carbonyle |

A partir de ces données, les structures chimiques des polyols polyesters synthétisés ont été déterminées par l'analyse des spectres obtenus (figures 1a à 1d et figures 2a à 2d).

*2.1.4 Analyse des résultats par RMN du phosphore 31*

L'analyse par résonnance magnétique du phosphore 31 de l'ensemble des polyols polyesters synthétisés permet de déterminer précisément les quantités de fonctions acides résiduelles dans les polyols polyesters, ainsi que la quantité de fonctions hydroxyles totales en s'affranchissant de la gêne stérique.

Tableau **2 : Indices d'hydroxyle et acide déterminés par analyses RMN-$^{31}$P**

| Polyol | Mmol $g^{-1}$ d'OH | IOH | Mmol $g^{-1}$ de COOH | IA |
|---|---|---|---|---|
| 1 | 10.37 | 581.8 | 0.60 | 33.7 |
| 2 | 11.21 | 628.9 | 0.40 | 22.4 |
| 3 | 10.52 | 590.2 | 0.42 | 48 |
| 4 | 9.03 | 506.6 | 0.33 | 18.5 |
| 5 | 8.24 | 462.3 | 0.4 | 22.4 |
| 6 | 7.84 | 439.8 | 0.22 | 12.3 |
| 7 | 8.7 | 488 | 0.20 | 11.22 |

*2.1.5 Structure des molécules obtenues*

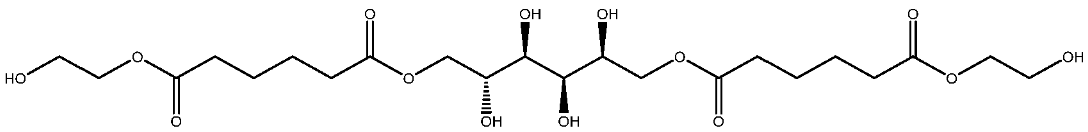

a: bis(1,2 éthanediol) -sorbitol diadipate **(526g/mol)**

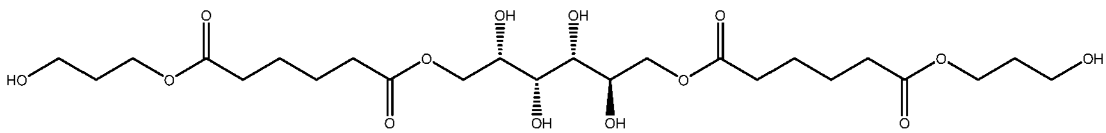

b: bis(1,3-propanediol) -sorbitol diadipate (554g/mol)

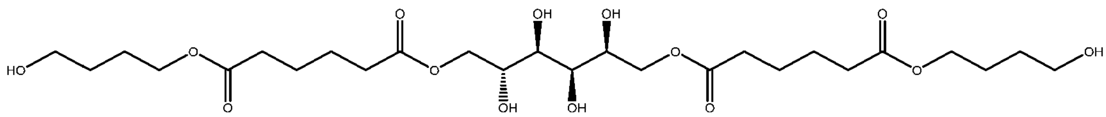

c: bis(1,4-butanediol) -sorbitol diadipate **(582g/mol)**

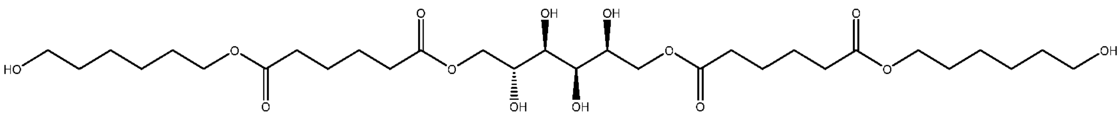

d: bis(1,6 hexanediol) -sorbitol diadipate **(638g/mol)**:

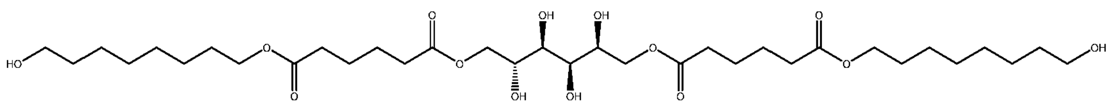

**e: bis(1,8 octanediol)-sorbitol diadipate (694g/mol):**

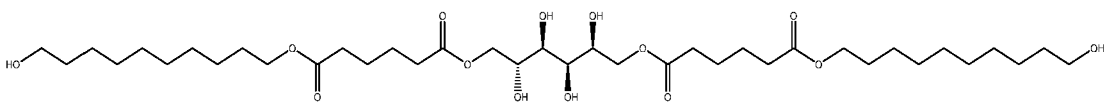

f: bis(1,10 décanediol)-sorbitol diadipate **(750g/mol)**:

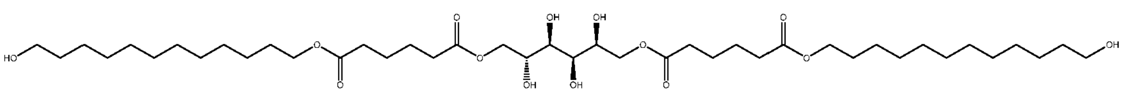

g: bis(1,12 dodécanediol) -sorbitol diadipate (806g/mol):
h: bis(1,4 butanediol)-sorbitol disuccinate (526g/mol):
i: sorbitol- diadipate- **disorbitol** (766g/mol):

Les caractérisations effectuées confirment que les polyols polyesters synthétisés ont une architecture bien définie en accord avec les protocoles opératoires en deux étapes employés visant à structurer les molécules. Les structures chimiques du **(a)** bis(1,2-éthanediol)-sorbitol diadipate (526g/mol), **(b)** bis(1,3-propanediol)-sorbitol diadipate (554g/mol), **(c)** bis(1,4-butanediol)-sorbitol diadipate (582g/mol), **(d)** bis(1,6-hexanediol)-sorbitol diadipate (638g/mol), **(e)** bis(1,8-octanediol)-sorbitol diadipate (694g/mol), **(f)** bis(1,10-décanediol)-sorbitol diadipate (750g/mol), **(g)** bis(1,12-dodécanediol)-sorbitol diadipate (806g/mol), **(h)** bis(1,4-butanediol)-sorbitol disuccinate (526g/mol), **(i)** sorbitol-diadipate-disorbitol (766g/mol). Les structures chimiques des polyols obtenus sont retrouvées ci-dessous avec la formule chimique générale $C_aH_bO_c$ avec 22 ≤ a ≤ 42, 38 ≤ b ≤ 78, 14 ≤ c≤ 22.

*2.2 Caractérisation des propriétés des polyols polyesters*

*2.2.1 Analyse DSC*

Les caractérisations thermiques de type DSC permettent de déterminer la cristallinité ou non du polyol polyester obtenu par la mesure d'une température de fusion (Tf) et d'une température de transition vitreuse (Tg). Le fait d'avoir des polyols polyesters amorphes (absence de Tf) avec une Tg basse (inférieure à la température ambiante) est particulièrement avantageux car cela assure que les polyols polyesters ne subiront pas de changement d'état lors de la formulation.

**Tableau 3 : Températures de transition vitreuse (Tg) et fusion (Tf) des polyols**

| Nature du polyol | Tg (°C) | Tf (°C) |
|---|---|---|
| bis(1,2-éthanediol)-sorbitol diadipate | -25 | / |
| bis(1,3-propanediol)-sorbitol diadipate | -27 | / |
| bis(1,4-butanediol)-sorbitol diadipate | -48 | / |
| bis(1,6-hexanediol)-sorbitol diadipate | -53 | / |
| bis(1,8-octanediol)-sorbitol diadipate | / | 23 |
| bis(1,10-décanediol)-sorbitol diadipate | -75 | 0-50 |
| bis(1,12-dodécanediol)-sorbitol diadipate | / | 30-40 |
| bis(1,4-butanediol)-sorbitol disuccinate | -40 | / |
| sorbitol-diadipate-sorbitol | -10 | / |

Ainsi, parmi l'ensemble des molécules synthétisées seulement trois présentent une température de fusion et sont donc sous forme cristalline : 1,8-octanediol-sorbitol adipate, le 1,10-décanediol-sorbitol adipate et le bis(1,12-dodécanediol)-sorbitol diadipate. Les autres polyols possèdent des Tg à des températures inférieures à 0°C (voir tableau 3).

Les faibles Tg et l'absence de températures de cristallisation et de fusion pour les trois polyols suivants: le bis(1,2-éthanediol)-sorbitol-diadipate, le bis(1,3-propanediol)-sorbitol-diadipate et le bis(1,4-butanediol)-sorbitol-diadipate permettent d'affirmer que ces polyols resteront des liquides visqueux à température ambiante et donc notamment aux températures d'utilisation des mousses de polyuréthanes, application préférée de l'invention (20-25°C). Les autres polyols ne présentant pas de Tf à savoir le bis(1,6-hexanediol)-sorbitol diadipate, le bis(1,4-butanediol)-sorbitol disuccinate et le sorbitol-diadipate-sorbtiol. Ils resteront visqueux (aspect cireux) à température ambiante (20-25°C). Les trois polyols présentant une Tf sont tous les deux solides à température ambiante, ce qui indique qu'une utilisation dans une autre application que le moussage tel que par exemple les revêtements, les adhésifs ou les élastomères sont recommandés. En effet, lors des opérations de moussage, le polyol doit pouvoir être mélangé à un polyisocyanate à 20-30°C, des produits cristallins avec des températures de fusion supérieures à 15°C ne sont pas recommandés. Ainsi, le bis(1,2-éthanediol)-sorbitol-diadipate, le bis(1,3-propanediol)-sorbitol-diadipate et le bis(1,4-butanediol)-sorbitol-diadipate sont particulièrement avantageux dans un large panel d'application et plus particulièrement le moussage. Les autres polyol polyesters pourront être destinés à des applications de type élastomère, adhésif ou revêtement.

*2.2.2 Analyse thermogravimétrique (ATG)*

L'ATG permet de déterminer les étapes de perte de masse et dégradation des polyols polyesters.

L'analyse des résultats montre une dégradation en deux temps. Ainsi, tous les polyols analysés se dégradent en deux étapes (voir Tableau 4).

**Tableau 4 : Températures de dégradations des polyols**

| Nature du polyol | 1ère dégradation | | 2ième dégradation |
|---|---|---|---|
| | Plage de T°C | %wt de perte | Plage de T°C |
| bis(1,2-éthanediol)-sorbitol diadipate | 50-130 | 7 | 150-450 |
| bis(1,3-propanediol)-sorbitol diadipate | 50-135 | 8 | 135-450 |
| bis(1,4-butanediol) -sorbitol diadipate | 40-130 | 7 | 130-450 |
| bis(1,6-hexanediol)-sorbitol diadipate | 40-162 | 11 | 162-450 |
| bis(1,8-octanediol)-sorbitol diadipate | 40-175 | 12 | 175-500 |
| bis(1,10-décanediol)-sorbitol diadipate | 40-200 | 16 | 200-450 |
| bis(1,12-dodécanediol)-sorbitol diadipate | 40-210 | 19 | 210-475 |
| bis(1,4-butanediol)-sorbitol disuccinate | 40-160 | 9 | 160-475 |
| sorbitol-adipate-sorbitol | 40-125 | 4 | 125-500 |

Lors de la formulation de mousses polyuréthanes, la réaction mise en jeu est exothermique et le milieu réactionnel atteint des températures de 100°C lors du procédé. Les polyols polyesters utilisés doivent donc être stables jusqu'à 100°C.

La première étape aux alentours de 100°C correspond à une perte de masse relative à la perte en eau résiduelle sous forme de vapeur et à la perte toujours sous forme de vapeur de monomères résiduels n'ayant pas réagi lors de la synthèse de la molécule. La perte de ces petites molécules n'est pas un inconvénient pour la formulation de mousses polyuréthanes puisqu'elles sont présentes en petites quantités et extrêmement réactives avec les isocyanates utilisées en formulation de mousses polyuréthanes. Elles auront donc réagi sans incident sur le reste du procédé avant évaporation avant que le milieu réactionnel n'atteigne 100°C.

La seconde dégradation correspond à la dégradation du polyol polyester. La dégradation majoritairement décrite dans la littérature est la scission de chaîne via l'atome d'hydrogène en $\beta$ de la liaison ester, ou $\alpha$ (phénomène moins important). Il y aura aussi des réactions intramoléculaires (backbiting) entrainant des cyclisations de chaînes ou de la cyclisation de molécules (issues d'une première dégradation par exemple) etc. Cela correspond à des températures auxquelles le polyol polyesters va se dégrader et perdre ses propriétés. Par conséquent, cette température est indicatrice de la stabilité du produit obtenu pour ces utilisations futures.

L'ensemble des polyols polyesters synthétisés sont stables au-delà de 100°C, ils sont donc tous utilisables pour la formulation d'une mousse, d'un élastomère, d'un adhésif ou d'un revêtement aux regards de leurs propriétés thermiques.

*2.2.3 Analyse rhéologiques des produits obtenus*

Les études rhéologiques (Tableau 5) permettent de déterminer la viscosité des polyols synthétisés en fonction de la température et leur caractère newtonien ou non en fonction du taux de cisaillement.

**Tableau 5 : Etude rhéologique**

| Polyol | Viscosité en fonction du taux de cisaillement à 20°C | | Viscosité en fonction du taux de cisaillement à 25°C | |
|---|---|---|---|---|
| | Viscosité (Pa.s) | Type de fluide | Viscosité (Pa.s) | Type de fluide |
| bis(1,3-propanediol)-sorbitol diadipate | 125,5 ±1,7 | Newtonien | 68,2 ±0,5 | Newtonien |
| bis(1,4-butanediol)-sorbitol diadipate | 44,6 ±1.2 | Newtonien | 24,3 ±0.2 | Newtonien |
| bis(1,6-hexanediol)-sorbitol diadipate | 48,7 ±1.2 | Newtonien | 27,16 ±1.7 | Newtonien |
| bis(1,4-butanediol)-sorbitol diadipate modifié glycérol | 5,3 ±0,4 | Newtonien | 3,4 ±0,2 | Newtonien |
| bis(1,4-butanediol)-sorbitol diadipate modifié ratio 2,1 en 1,4 BDO | 12,2 ±1,5 | Newtonien | 7,3 ±0.5 | Newtonien |

Ces résultats montrent que les produits obtenus à savoir le bis(1,4-butanediol)-sorbitol-diadipate, le bis(1,6-hexanediol)-sorbitol-diadipate et les bis(1,4-butanediol)-sorbitol-diadipate modifié glycérol et ratio 2,1 en 1,4BDO ont des caractéristiques particulièrement avantageuses pour une utilisation dans l'ensemble des applications utilisant classiquement des produits pétro-sourcés telles que les mousses, les élastomères, les adhésifs ou revêtements de polyuréthanes du fait de leur viscosité suffisamment basse (caractère liquide) permettant leur mélange à température ambiante avec les autres composants de la formulation. Le bis(1,3-propanediol)-sorbitol diadipate présente une viscosité élevée à 20°C, néanmoins, sa baisse de viscosité à 25°C pourrait permettre l'utilisation de ce polyol polyester à cette température dans de telles formulations. Les autres polyols polyesters n'ont pas été soumis à des analyses de viscosités à 20°C et 25°C car ils sont cireux (solide) à ces températures (voir Tableau 3) ce qui rend leur utilisation en formulation de mousses polyuréthanes moins intéressante mais est sans incidence pour des formulations d'élastomères, d'adhésifs ou de revêtements puisque le mélange de la formulation peut se faire à une température supérieure à la température ambiante.

*2.2.4 Analyse de l'indice hydroxyles (IOH)*

La mesure de l'indice hydroxyles est utilisée notamment pour l'élaboration de mousses polyuréthanes. Elle permet d'évaluer la part d'isocyanate en formulation de mousse polyuréthane.

La synthèse de polyols comprenant de nombreux groupements hydroxyles de nature différente (primaires et secondaires) rend la titration difficile en termes de gêne stérique pour les groupes hydroxyles secondaires placés côte à côte sur les groupements sorbitol. Ainsi les indices hydroxyles (IOH) présentés dans le Tableau 6 reflètent le nombre d'hydroxyles accessibles et pris en compte pour la formulation de mousse polyuréthane.

**Tableau 6 : IOH des polyols synthétisés**

| Nature du polyol | IOH (mg KOH/g) |
|---|---|
| bis(1,4-butanediol)-sorbitol disuccinate | 409 |
| sorbitol-diadipate-sorbitol | 692 |
| bis(1,2-éthanediol)-sorbitol diadipate | 533 |
| bis(1,3-propanediol)-sorbitol diadipate | 437 |
| bis(1,4-butanediol)-sorbitol diadipate | 400 |
| bis(1,6-hexanediol)-sorbitol diadipate | 415 |
| bis(1,8-octanediol)-sorbitol diadipate | 355 |
| bis(1,10-décanediol)-sorbitol diadipate | 326 |
| bis(1,12-dodécanediol)-sorbitol diadipate | 311 |

Les gammes d'indices d'hydroxyles recherchées par un fabricant de mousse dépend du type de mousses (PUR, PIR, etc..) et du nombre de polyols introduits dans la formulation. En général, pour une mousse rigide, la gamme s'étend de 100 à 700 mg KOH/g. Dans le cas d'une mousse de type PUR la gamme d'indice d'hydroxyles (IOH) permettant l'obtention d'un réseau tridimensionnel réticulé est compris entre 300 et 700 mg KOH/g alors que pour un réseau de type PIR, la gamme d'IOH doit être comprise entre 100 et 500 mg KOH/g. Ainsi l'ensemble des polyols polyesters présentent des indices d'hydroxyles permettant leur utilisation en formulation PUR et PIR.

*2.2.5 Titration acide des polyols*

La titration acide des polyols incorporés dans les mousses polyuréthanes rigides est utilisée dans la formulation de ces mousses car les fonctions acides résiduelles des polyols polyesters sont susceptibles d'inhiber les catalyseurs utilisés lors de la formulation de mousses polyuréthanes rigides si leur quantité est trop importante. Les étapes de titration et les trois types de neutralisations ont été testés sur le bis(1,4-butanediol)-sorbitol diadipate car l'ensemble des caractérisations précédentes en font le polyol polyester le plus adapté à la formulation de mousses polyuréthanes notamment des mousses polyuréthanes rigides.

La titration avant et après neutralisation permet de juger de l'efficacité de cette dernière. L'étape de neutralisation permet de diminuer l'indice d'acide par quatre voir dix en conditions optimales.

On remarquera différents points lors des neutralisations avec les bases. Avec la soude, le polyol polyester final est blanchâtre alors qu'avec la potasse, on peut extraire un précipité acido-basique solide et l'aspect du polyol polyester n'a pas changé. Les précipités peuvent toujours être condensés par centrifugation, mais la neutralisation avec la potasse

présente un avantage certain en facilitant l'élimination du complexe potasse/acide qui précipite en agglomérats de taille plus élevée que ceux de la soude.

**Tableau 7 : Indice d'acidité avant et après neutralisation**

| | Après synthèse | Neutralisé NaOH | Neutralisé KOH | Neutralisé hexanol |
|---|---|---|---|---|
| Indice d'acide (mg KOH/g) | 59 ±2.3 | 8,3 ±0.4 | 15,9 ±1.6 | 24,2 ±2 |

L'indice d'acidité du polyol polyester est évalué avant et après neutralisation L'ensemble des résultats pour le *bis(1,4-butanediol)-sorbitol* diadipate sont présent dans le tableau 7. L'indice d'acidité correspond au nombre de mg de KOH nécessaire pour neutraliser l'ensemble des groupements acides carboxyliques présent dans un gramme d'un polyol polyester. L'indice d'acidité peut être déterminé par dosage colorimétrique au bleu de méthylène en utilisant une solution de potasse à 0.1 mol/L dans le méthanol.

Les trois types de neutralisation permettent d'abaisser significativement l'indice d'acidité du polyol polyester. Cette neutralisation permet l'obtention d'une formulation de mousse polyuréthane rigide de très bonne qualité.

**Exemple 3 : Formulation de mousses polyuréthane**

Des mousses de polyuréthane ont été obtenues en utilisant les polyols polyester à base de bis(1,4-butanediol)-sorbitol-diadipate et bis(1,4-butanediol)-sorbitol-diadipate neutralisé. Un prémix contenant le polyol et les différents additifs et gaz d'expansion est préparé puis la quantité nécessaire de diisocyante polymérique (pMDI) y est ajoutée (voir Tableau 8). Le pré-mix est obtenu par un ajout successif des composants entre lesquels une étape d'homogénéisation est effectuée.

**Tableau 8 : Formulation de mousses PUR rigides**

| Référence Fournisseur | Témoin PUR | Polyol non neut PUR | Neut KOH 2 parts Cat. aminé | Neut Hexanol distillé PUR | Neut NaOH PUR | Neut Hexanol PUR |
|---|---|---|---|---|---|---|
| | 114,4 | 115,0 | 115,0 | 115,0 | 115,0 | 115,0 |
| pMDI | 190,14 | 147,38 | 147,29 | 147,29 | 147,29 | 147,29 |
| Polyol témoin | 100,00 | | | | | |
| Polyol base sorbitol | | 100 | | | | |
| Polyol base sorbitol neut. NaOH | | | | | 100,00 | |
| Polyol base sorbitol neut. KOH | | | 100,00 | | | |
| Polyol base sorbitol neut. Hexanol | | | | | | 100,00 |
| Polyol base sorbitol neut. hexanol distillé | | | | 100,00 | | |
| Eau | 1,66 | 1,6 | 1,60 | 1,60 | 1,60 | 1,60 |
| Surfactant polyether polysiloxane B1048 | 2,50 | 2,5 | 2,56 | 2,56 | 2,56 | 2,50 |
| Catalyseur : Diméthylecyclohex yleamine | 2,35 | 1,7 | 2,00 | 2,06 | 2,06 | 2,11 |
| Agent ignifugeant : TCPP | 10,06 | 10 | 10,00 | 10,00 | 10,05 | 10,05 |
| Agent gonflant : Isopentane | 15,37 | 11,92 | 13,08 | 13,52 | 13,96 | 15,73 |

(suite)

| Référence Fournisseur | Témoin PUR | Polyol non neut PUR | Neut KOH 2 parts Cat. aminé | Neut Hexanol distillé PUR | Neut NaOH PUR | Neut Hexanol PUR |
|---|---|---|---|---|---|---|
| TOTAL | 322,08 | 275,10 | 276,53 | 277,03 | 277,52 | 279,28 |

Dans le cadre de formulations de mousses polyuréthanes (PUR) rigides quatre essais ont été conduits : un témoin industriel (polyéther standard de fonctionnalité 3,3 et un indice d'hydroxyle de 585 mg KOH/g), une formulation avec le polyol polyester à base de 1,4-butanediol-sorbitol-adipate neutralisé au KOH, puis NaOH et neutralisé à l'hexanol. En effet, ce polyol polyester présente la meilleure viscosité à température ambiante et un faible coût de production (monomères peu coûteux et biosourcés ou biosourçables) pour la formulation de mousses PUR.

Les formulations de prémix nécessaires pour effectuer les mesures des temps caractéristiques sont exprimées en nombre de parts (Tableau 8). Au prémix est ensuite ajoutée la quantité de polyisocyanate (type 4,4 MDI polymérique) souhaitée (Tableau 8).

Les temps caractéristiques de l'obtention d'une mousse sont mesurés à savoir le temps de crème, de fils et hors-poisse ainsi que la hauteur de mousse (Tableau 9).

**Tableau 9 : Caractéristiques de la mousse obtenue**

| Temps caractéristiques en s | Témoin PUR | Polyol non neut. PUR | Neut. KOH 2 parts Catalyseur aminé | Neut. Hexanol distillé PUR | Neut. NaOH PUR | Neut. Hexanol PUR |
|---|---|---|---|---|---|---|
| Crème | 10 | 75 | 15 | 50 | 14 | 45 |
| Fil | 39 | 420 | 70 | 163 | 73 | 203 |
| Hors-poisse | 60 | 1500 | 115 | 299 | 80 | 346 |
| Hauteur mousse | 26,1 | 5 | 23,7 | 21,8 | 23,4 | 22,6 |

Les temps caractéristiques obtenus avec les polyols polyesters neutralisés à la potasse et à la soude sont satisfaisants, dans le cas des polyols polyesters neutralisés avec de l'hexanol les temps sont moins avantageux. Ainsi, l'utilisation du polyol polyester neutralisé, permet comparativement aux polyols polyesters non neutralisés, l'observation d'une meilleure cinétique de réaction (plus courte) et une hauteur de mousse particulièrement avantageuse, équivalente aux produits non biosourcés (témoin PUR). Il est donc recommandé d'utiliser une version neutralisée du polyol polyester pour la formulation de mousses polyuréthane rigides.

A l'aide d'un polyol polyester à base de sorbitol neutralisé, il est donc possible de formuler une mousse polyuréthane avec des caractéristiques cinétiques similaires à celles des produits non biosourcés actuellement utilisés. Les mousses de meilleures qualités sont obtenues à partir de polyols neutralisés à la potasse ou à la soude.

## Revendications

**1.** Polyol polyester de formule générale **Rx-Ry-Z-Ry'-Rx'** dans laquelle :

- **Z** est un sucre alcool en C3 à C8,
- **Ry** et **Ry'** sont des diesters de formule $-OOC-C_n-COO-$ avec **n** compris entre 2 et 34, et
- **Rx** et **Rx**' sont des monoalcools identiques ou différents en C2 à C12.

**2.** Polyol polyester selon la revendication **1, caractérisé en ce que** le sucre alcool **Z** est choisi parmi le glycérol, le sorbitol, l'érythritol, le xylitol, l'arabitol, le ribitol, le dulcitol, le mannitol et le volémitol.

**3.** Polyol polyester selon la revendication 1 ou la revendication 2, **caractérisé en ce que** Z est un sucre alcool en C4 à C7.

**4.** Polyol polyester selon l'une quelconque des revendications 1 à 3, **caractérisé en ce que n** est compris entre 3 et 22.

**5.** Polyol polyester selon l'une quelconque des revendications 1 à 4, **caractérisé en ce que** Rx et Rx' sont des

monoalcools identiques ou différents en C3 à C8.

**6.** Procédé d'obtention d'un polyol polyester selon l'une quelconque des revendications **1** à **5,** comprenant les étapes suivantes :

a) une étape de polycondensation à une température comprise entre 110 et 200°C, avantageusement durant 5 à 10 heures :

i. d'un sucre alcool **Z** en C3 à C8, préférentiellement en C4 à C7,
ii. de deux diacides **Y** et **Y'** en C4 à C36 identiques ou différents, préférentiellement C5 à C24,
iii. de deux diols **X** et **X'** en C2 à C12 identiques ou différents, préférentiellement en C3 à C8,

ladite étape de polycondensation comprenant :

- une première polycondensation (a) du sucre alcool **Z** et des diacides **Y** et **Y'** et
- une seconde polycondensation (b) du produit obtenu en (a) avec les diols **X** et **X'** ; et

b) optionnellement, une étape de neutralisation.

**7.** Procédé selon la revendication **6, caractérisé en ce que** le sucre alcool **Z** est choisi parmi le glycérol, le sorbitol, l'érythritol, le xylitol, l'arabitol, le ribitol, le dulcitol, le mannitol et le volémitol.

**8.** Procédé selon la revendication **6** ou la revendication **7, caractérisé en ce que** les diacides **Y** et **Y'** sont indépendamment choisis parmi l'acide butanedioïque, l'acide pentanedioïque, l'acide hexanedioïque, l'acide heptanedioïque, l'acide octanedioïque, l'acide nonanedioïque, l'acide décanedioïque, l'acide undécanedioïque, l'acide dodécanedioïque, l'acide tridécanedioïque, l'acide tétradécanedioïque, l'acide pentadécanedioïque, l'acide hexadécanedioïque et leur mélange.

**9.** Procédé selon l'une quelconque des revendications **6** à **8, caractérisé en ce que** les diols **X** et **X'** sont indépendamment choisis parmi le 1,2-éthanediol, le 1,3-propanediol, le 1,4-butanediol, le 1,6-hexanediol, le 1,8-octanediol, le 1,10-décanediol, le 1,12-dodécanediol et leur mélange.

**10.** Polymère comprenant le polyol polyester selon l'une quelconque des revendications 1 à 5, ledit polymère étant un polyuréthane et/ou un polyisocyanurate.

**11.** Polymère selon la revendication **10,** ledit polymère présentant un taux de polymérisation de 2 à 10 et/ou une masse molaire comprise entre 1000 g/mol et 10 000 g/mol, la masse molaire étant déterminée selon la méthode indiquée dans la description.

**12.** Composition comprenant le polyol polyester selon l'une quelconque des revendications **1** à **5** ou le polymère selon la revendication 10 ou la revendication **11.**

**13.** Composition selon la revendication **12, caractérisée en ce qu'**il s'agit d'une mousse, d'un élastomère, d'un adhésif, d'un revêtement ou d'une composition permettant l'obtention de l'un quelconque des mousse, élastomère, adhésif et revêtement après polymérisation.

**14.** Composition selon la revendication **12** ou la revendication **13** comprenant en outre un catalyseur de réaction et un polyisocyanate.

**15.** Procédé d'obtention d'une mousse, d'un revêtement, d'un adhésif ou d'un élastomère de polyuréthane ou de polyisocyanurate comprenant :

- une étape d'obtention d'un polyol polyester selon l'une quelconque des revendications **1** à **5** ou d'un polymère selon la revendication **10** ou la revendication **11,** ledit polymère présentant un taux de polymérisation de 2 à 10, et de préférence une masse molaire comprise entre 1000 g/mol et 10 000 g/mol, la masse molaire étant déterminée selon la méthode indiquée dans la description,
- une étape d'addition d'au moins un polyisocyanate et d'au moins un catalyseur de réaction, et
- une étape de polymérisation.

16. Utilisation d'un polyol polyester selon l'une quelconque des revendications 1 à 5 ou d'un polymère selon la revendication **10** ou la revendication **11** dans la fabrication d'une mousse, d'un revêtement, d'un adhésif ou d'un élastomère de polyuréthane ou de polyisocyanurate.

## Patentansprüche

1. Polyesterpolyol der allgemeinen Formel **Rx-Ry-Z-Ry'-Rx'**, in der:

   - **Z** ein C3- bis C8-Zuckeralkohol ist,
   - **Ry** und **Ry'** Diester der Formel -OOC-$C_n$-COO- sind, wobei **n** zwischen 2 und 34 liegt, und
   - **Rx** und **Rx'** gleiche oder verschiedene C2- bis C12-Monoalkohole sind.

2. Polyesterpolyol nach Anspruch **1, dadurch gekennzeichnet, dass** der Zuckeralkohol **Z** aus Glycerin, Sorbit, Erythrit, Xylit, Arabit, Ribit, Dulcit, Mannit und Volemit ausgewählt ist.

3. Polyesterpolyol nach Anspruch **1** oder Anspruch **2, dadurch gekennzeichnet, dass Z** ein C4- bis C7-Zuckeralkohol ist.

4. Polyesterpolyol nach einem der Ansprüche **1** bis **3, dadurch gekennzeichnet, dass n** zwischen 3 und 22 liegt.

5. Polyesterpolyol nach einem der Ansprüche **1** bis **4, dadurch gekennzeichnet, dass Rx** und **Rx**' gleiche oder verschiedene C3- bis C8-Monoalkohole sind.

6. Verfahren zum Erhalten eines Polyesterpolyols nach einem der Ansprüche **1** bis **5,** umfassend die folgenden Schritte:

   a) einen Schritt einer Polykondensation bei einer Temperatur, die zwischen 110 und 200 °C liegt, auf vorteilhafte Weise während 5 bis 10 Stunden, von:

      i. einem C3- bis C8-, vorzugsweise C4- bis C7-Zuckeralkohol **Z,**
      ii. zwei gleichen oder verschiedenen C4- bis C36-, vorzugsweise C5-bis C24-Disäuren **Y** und **Y'**,
      iii. zwei gleichen oder verschiedenen C2- bis C12-, vorzugsweise C3-bis C8-Diolen **X** und **X',**

   wobei der Schritt der Polykondensation umfasst:

      - eine erste Polykondensation (a) des Zuckeralkohols **Z** und der Disäuren **Y** und **Y'** und
      - eine zweite Polykondensation (b) des in (a) erhaltenen Produkts mit den Diolen **X** und **X'**; und

   b) optional einen Schritt einer Neutralisation.

7. Verfahren nach Anspruch **6, dadurch gekennzeichnet, dass** der Zuckeralkohol **Z** aus Glycerin, Sorbit, Erythrit, Xylit, Arabit, Ribit, Dulcit, Mannit und Volemit ausgewählt ist.

8. Verfahren nach Anspruch 6 oder Anspruch 7, **dadurch gekennzeichnet, dass** die Disäuren **Y** und **Y'** unabhängig aus Butandisäure, Pentandisäure, Hexandisäure, Heptandisäure, Octandisäure, Nonandisäure, Decandisäure, Undecandisäure, Dodecandisäure, Tridecandisäure, Tetradecandisäure, Pentadecandisäure, Hexadecandisäure und deren Gemisch ausgewählt sind.

9. Verfahren nach einem der Ansprüche 6 bis 8, **dadurch gekennzeichnet, dass** die Diole **X** und **X'** unabhängig aus 1,2-Ethandiol, 1,3-Propandiol, 1,4-Butandiol, 1,6-Hexandiol, 1,8-Octandiol, 1,10-Decandiol, 1,12-Dodecandiol und deren Gemisch ausgewählt sind.

10. Polymer, umfassend das Polyesterpolyol nach einem der Ansprüche **1** bis **5,** wobei das Polymer ein Polyurethan und/oder ein Polyisocyanurat ist.

11. Polymer nach Anspruch **10,** wobei das Polymer einen Polymerisationsgrad von 2 bis 10 und/oder eine Molmasse, die zwischen 1000 g/mol und 10.000 g/mol liegt, aufweist, wobei die Molmasse gemäß dem in der Beschreibung angegebenen Methode bestimmt wird.

**12.** Zusammensetzung, umfassend das Polyesterpolyol nach einem der Ansprüche 1 bis 5 oder das Polymer nach Anspruch **10** oder Anspruch **11.**

**13.** Zusammensetzung nach Anspruch **12, dadurch gekennzeichnet, dass** es sich um einen Schaumstoff, ein Elastomer, einen Klebstoff, eine Beschichtung oder eine Zusammensetzung, die das Erhalten eines bzw. einer beliebigen von einem Schaumstoff, einem Elastomer, einem Klebstoff und einer Beschichtung nach Polymerisation ermöglicht, handelt.

**14.** Zusammensetzung nach Anspruch **12** oder Anspruch **13,** weiterhin umfassend einen Reaktionskatalysator und ein Polyisocyanat.

**15.** Verfahren zum Erhalten eines Schaumstoffs, einer Beschichtung, eines Klebstoffs oder eines Elastomers aus Polyurethan oder Polyisocyanurat, umfassend:

- einen Schritt eines Erhaltens eines Polyesterpolyols nach einem der Ansprüche **1** bis **5** oder eines Polymers nach Anspruch **10** oder Anspruch **11,** wobei das Polymer einen Polymerisationsgrad von 2 bis 10 und vorzugsweise eine Molmasse, die zwischen 1000 g/mol und 10.000 g/mol liegt, aufweist, wobei die Molmasse gemäß der in der Beschreibung angegebenen Methode bestimmt wird,
- einen Schritt einer Zugabe von mindestens einem Polyisocyanat und mindestens einem Reaktionskatalysator, und
- einen Schritt einer Polymerisation.

**16.** Verwendung eines Polyesterpolyols nach einem der Ansprüche 1 bis 5 oder eines Polymers nach Anspruch 10 oder Anspruch 11 bei der Herstellung eines Schaumstoffs, einer Beschichtung, eines Klebstoffs oder eines Elastomers aus Polyurethan oder Polyisocyanurat.

## Claims

**1.** Polyester polyol of general formula **Rx-Ry-Z-Ry'-Rx',** wherein:

- **Z** is a C3 to C8 sugar alcohol,
- **Ry** et **Ry'** are diesters of formula -OOC-$C_n$-COO- with **n** ranging from 2 to 34, and
- **Rx** et **Rx'** are identical or different C2 to C12 monoalcohols.

**2.** Polyester polyol according to claim **1, characterised in that** the sugar alcohol **Z** is chosen from glycerol, sorbitol, erythritol, xylitol, arabitol, ribitol, dulcitol, mannitol and volemitol.

**3.** Polyester polyol according to claim **1** or claim **2, characterised in that Z** is a C4 to C7 sugar alcohol.

**4.** Polyester polyol according to any one of claims **1** to **3**, **characterised in that n** is ranging from 3 to 22.

**5.** Polyester polyol according to any one of claims **1** to **4, characterised in that Rx** and **Rx'** are identical or different C3 to C8 monoalcohols.

**6.** Process for obtaining a polyester polyol according to any one of claims **1** to **5,** comprising the following steps:

a) a polycondensation step at a temperature ranging from 110 to 200°C, advantageously for 5 to 10 hours:

i. of a C3 to C8, preferably C4 to C7, sugar alcohol **Z,**
ii. of two identical or different C4 to C36, preferably C5 to C24, diacids **Y** and **Y',**
iii. of two identical or different C2 to C12, preferably C3 to C8, diols X and X',

said polycondensation step comprising:

- a first polycondensation (a) of the sugar alcohol **Z** and of the diacids **Y** and **Y' and**
- a second polycondensation (b) of the product obtained in (a) with the diols **X** and **X';** and

b) optionally, a neutralization step.

**7.** Process according to claim **6, characterised in that** the sugar alcohol **Z** is chosen from glycerol, sorbitol, erythritol, xylitol, arabitol, ribitol, dulcitol, mannitol and volemitol.

**8.** Process according to claim 6 or claim 7, **characterised in that** the diacids Y and Y' are independently chosen from butanedioic acid, pentanedioic acid, hexanedioic acid, heptanedioic acid, octanedioic acid, nonanedioic acid, decanedioic acid, undecanedioic acid, dodecanedioic acid, tridecanedioic acid, tetradecanedioic acid, pentadecanedioic acid, hexadecanedioic acid and a mixture thereof.

**9.** Process according to any one of claims 6 to 8, **characterised in that** the diols X and X' are independently chosen from 1,2-ethanediol, 1,3-propanediol, 1,4-butanediol, 1,6-hexanediol, 1,8-octanediol, 1,10-decanediol, 1,12-dodecanediol and a mixture thereof.

**10.** Polymer comprising the polyester polyol according to any one of claims **1** to **5,** said polymer being a polyurethane and/or a polyisocyanurate.

**11.** Polymer according to claim **10,** said polymer presenting a polymerization rate from 2 to 10 and/or a molar mass ranging from 1000 g/mol to 10 000 g/mol, the molar mass being determined according to the method indicated in the description.

**12.** Composition comprising the polyester polyol according to any one of claims **1** to **5** or the polymer according to claim **10** or claim **11.**

**13.** Composition according to claim **12, characterised in that** it is a foam, an elastomer, an adhesive, a coating or a composition allowing for the obtaining of any one of foam, elastomer, adhesive and coating after polymerisation.

**14.** Composition according to claim **12** or claim **13,** further comprising a reaction catalyst and a polyisocyanate.

**15.** Process for obtaining a polyurethane or polyisocyanurate foam, coating, adhesive or elastomer comprising:

- a step of obtaining a polyester polyol according to any one of claims **1** to **5** or a polymer according to claim **10** or claim **11,** said polymer having a polymerization rate from 2 to 10, and preferably a molar mass ranging from 1000 g/mol to 10 000 g/mol, the molar mass being determined according to the method indicated in the description,
- a step of adding at least one polyisocyanate and at least one reaction catalyst, and
- a step of polymerisation.

**16.** Use of a polyester polyol according to any one of claims **1** to **5** or of a polymer according to claim **10** or claim **11** in the manufacture of a polyurethane or polyisocyanurate foam, coating, adhesive or elastomer.

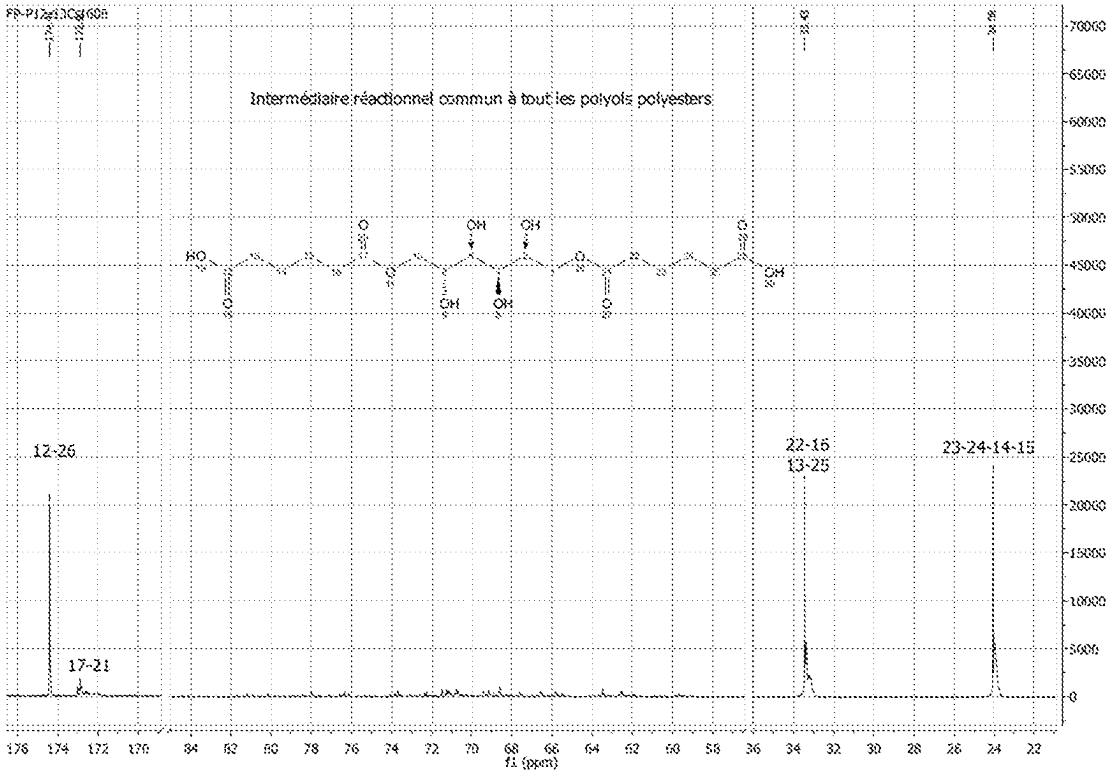
Intermédiaire réactionnel commun à tout les polyols polyesters
12-26
17-21
22-16
13-25
23-24-14-15
f1 (ppm)

**Fig. 1a**

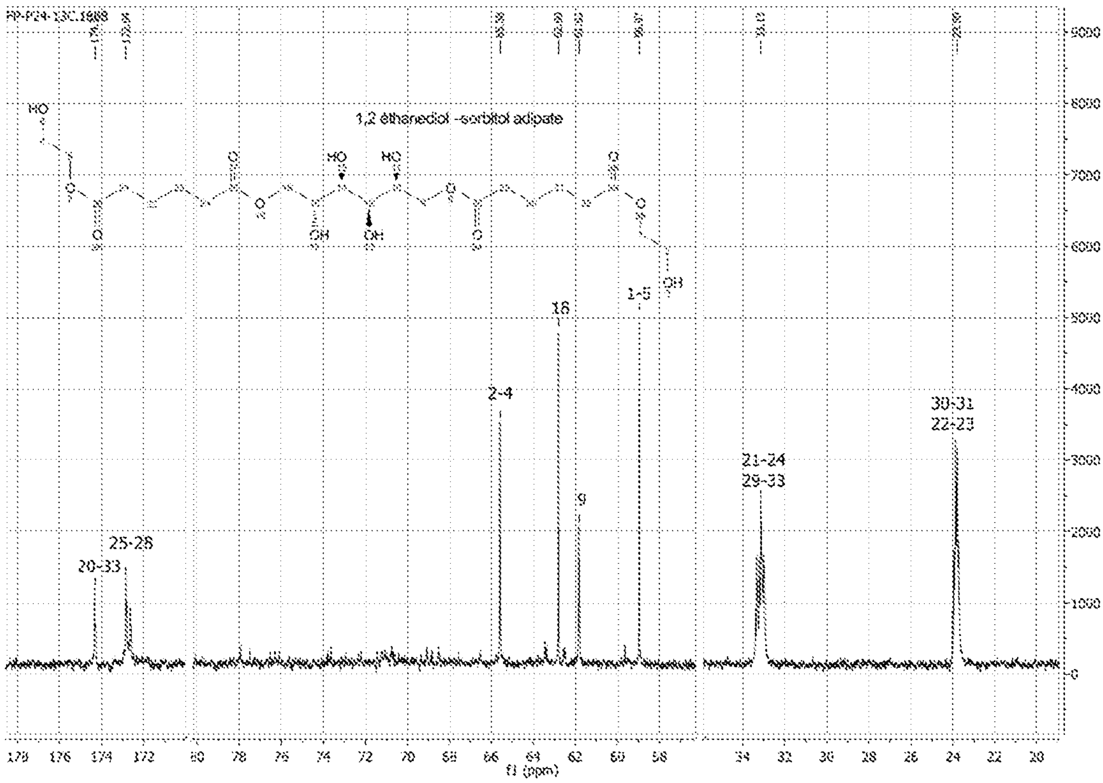
1,2 éthanediol -sorbitol adipate
20-33
25-28
2-4
16
9
1-5
21-24
29-33
30-31
22-23
f1 (ppm)

**Fig. 1b**

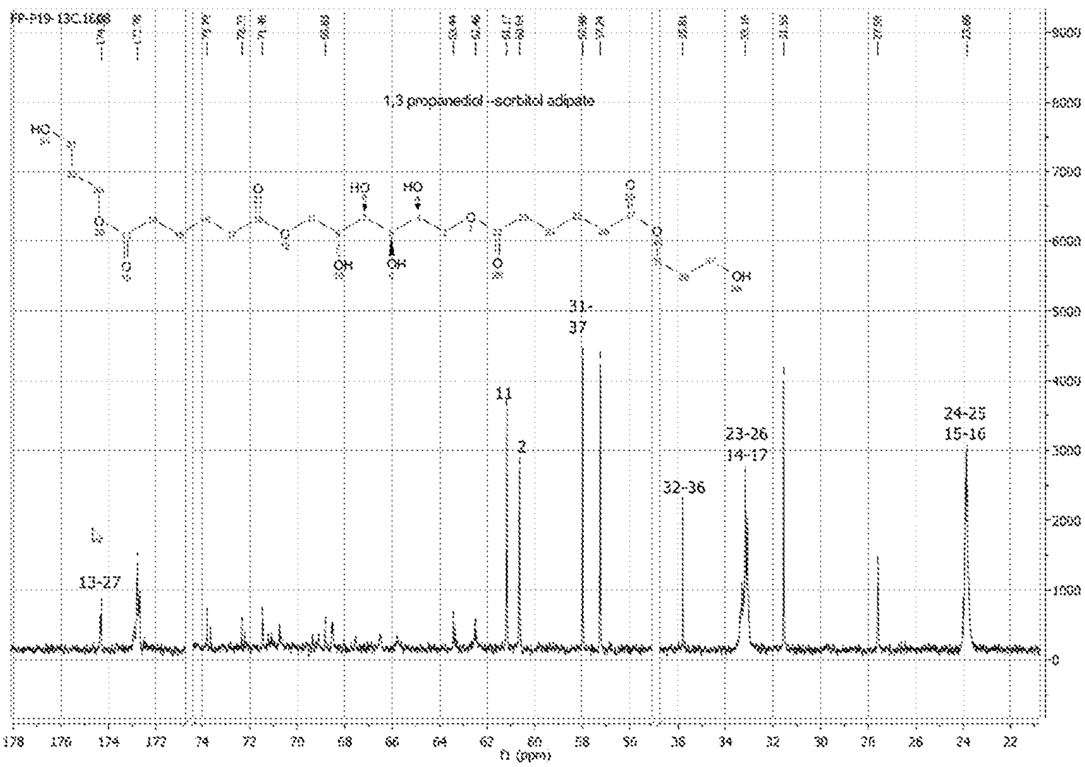
1,3 propanediol -sorbitol adipate
13-27
11
2
31-37
32-36
23-26
14-17
24-25
15-16
f1 (ppm)

Fig. 1c

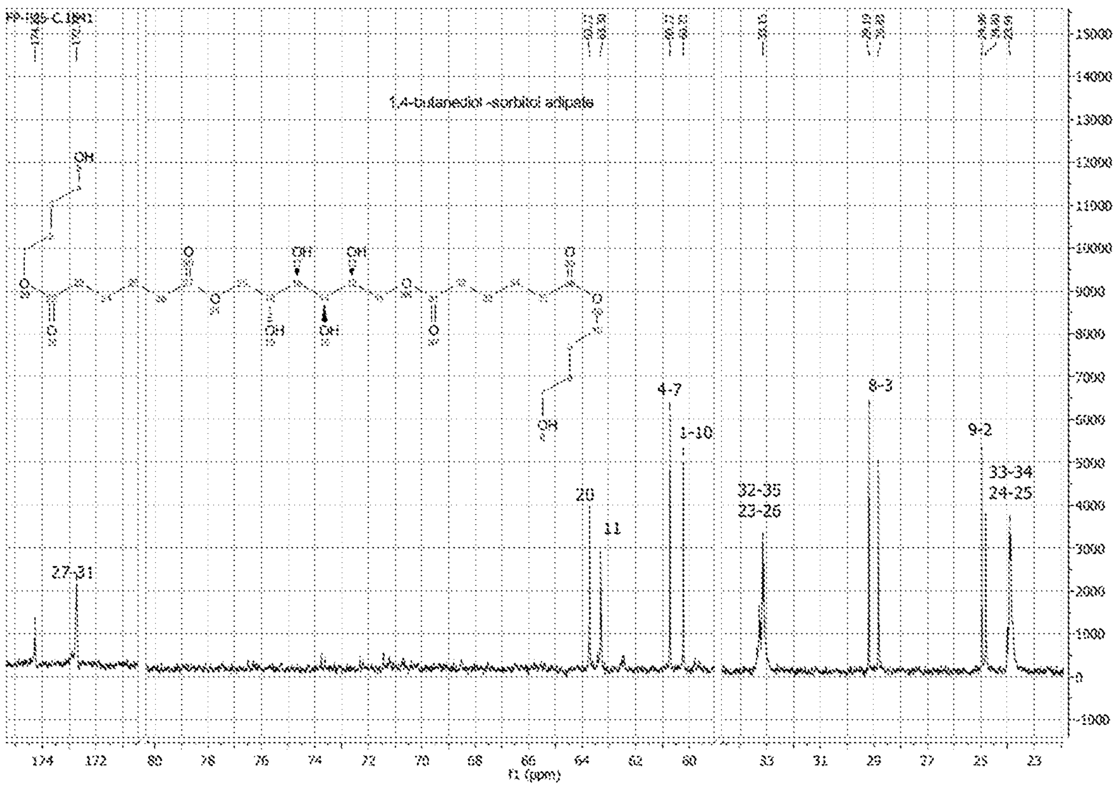
1,4-butanediol -sorbitol adipate
27-31
20
11
4-7
1-10
32-35
23-26
8-3
9-2
33-34
24-25
f1 (ppm)

Fig. 1d

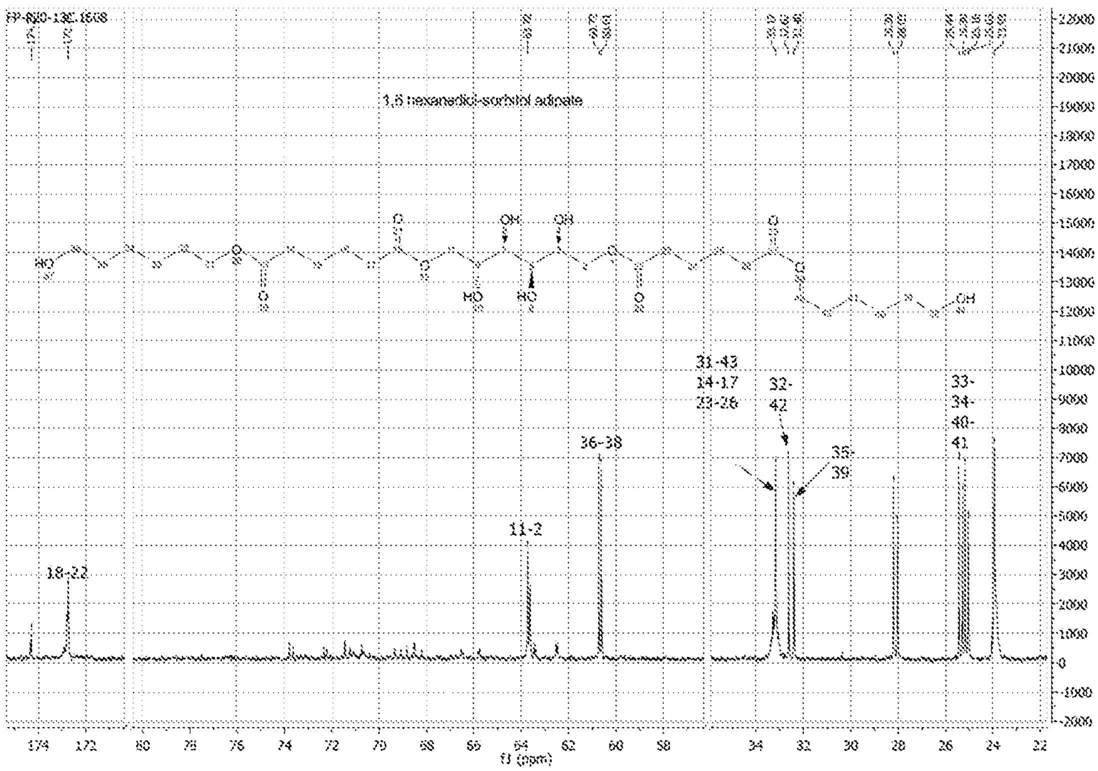

Fig. 2a

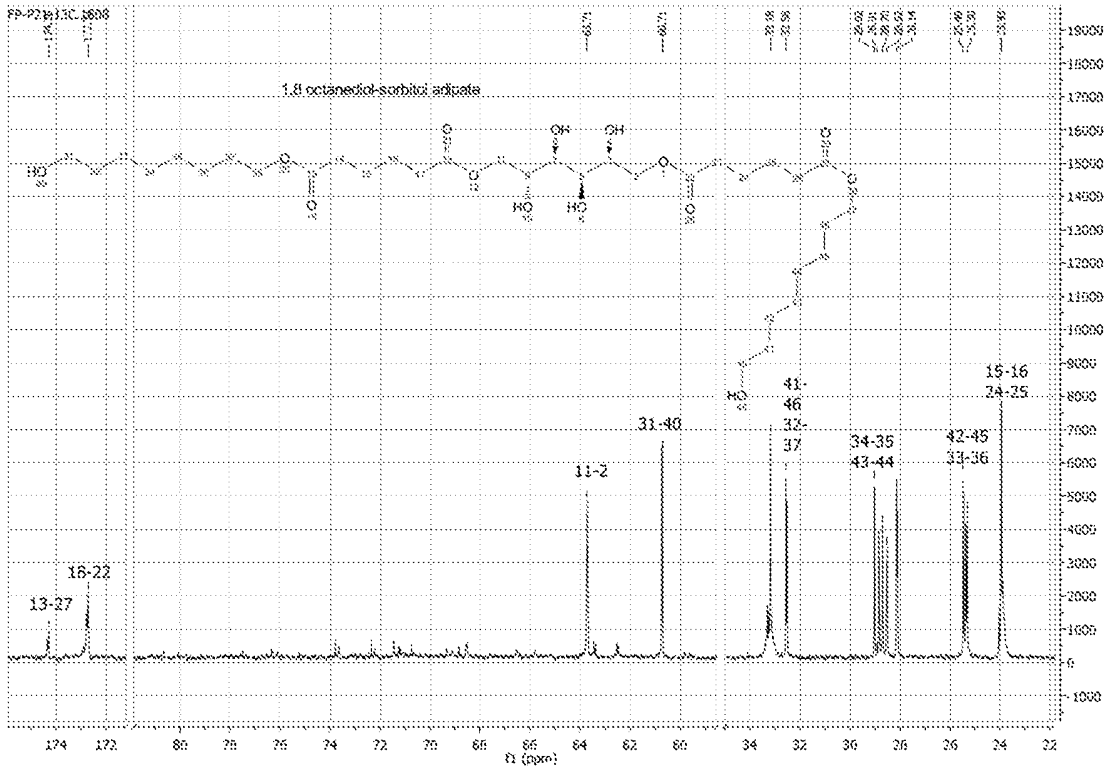

Fig. 2b

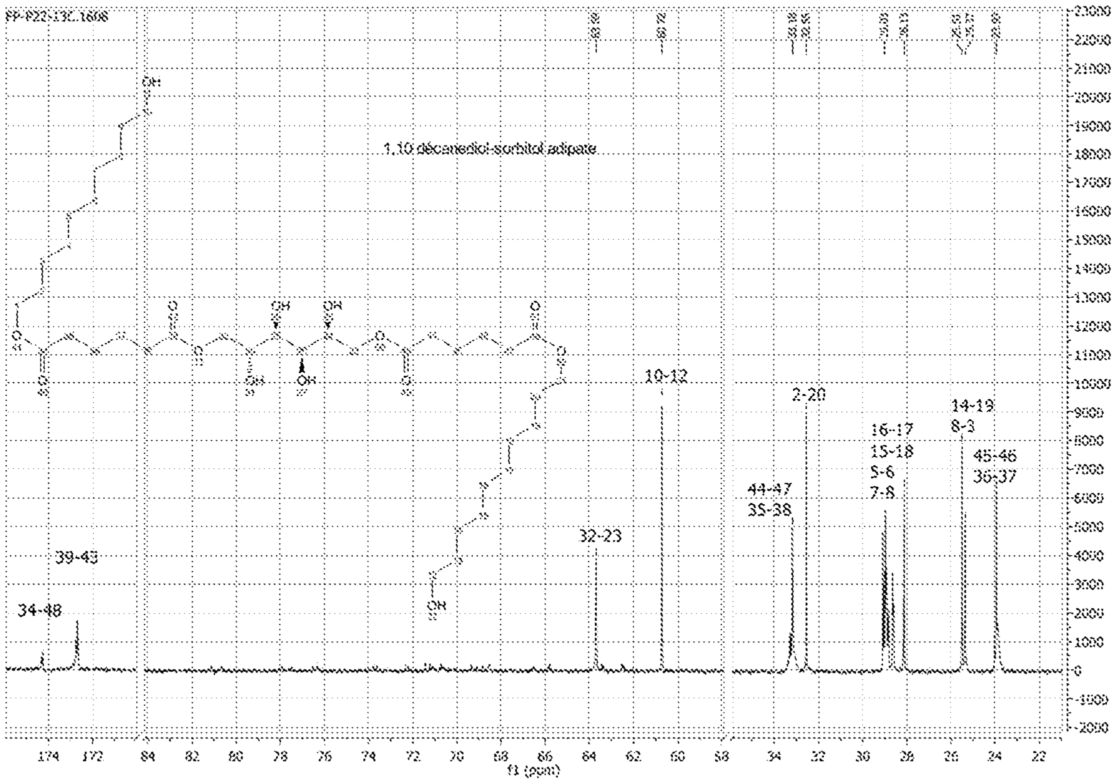
1,10 décanediol-sorbitol adipate
10-12
2-20
14-19
8-3
16-17
15-18
5-6
7-8
45-46
36-37
44-47
35-38
32-23
39-43
34-48
f1 (ppm)

**Fig. 2c**

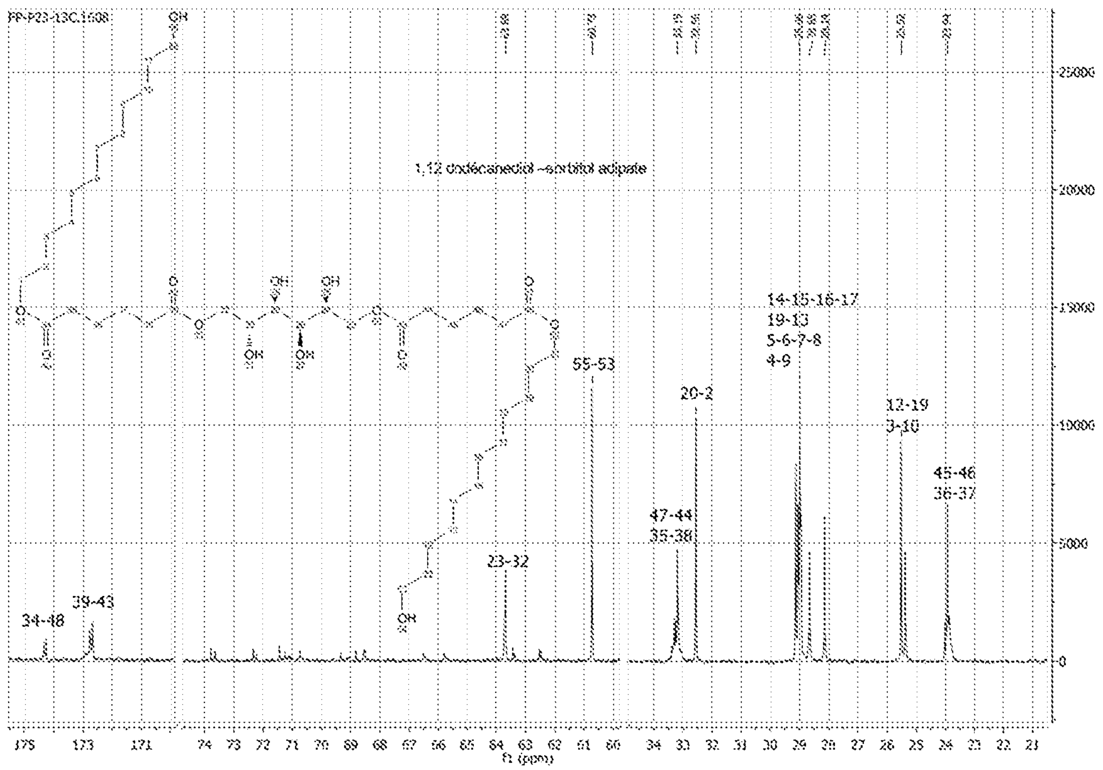
1,12 dodécanediol -sorbitol adipate
14-15-16-17
19-13
5-6-7-8
4-9
55-53
20-2
12-19
3-10
45-46
36-37
47-44
35-38
23-32
39-43
34-48
f1 (ppm)

**Fig. 2d**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 2605233 A
- US 20140200327 A
- FR 2987840
- US 4404295 A


### Littérature non-brevet citée dans la description


- *CHEMICAL ABSTRACTS,* 50-70-4
- **JOSEPH E. WILSON ; RAYMOND H. FOWLER.** Rigid Urethanes Foams Based on Sorbitol Derivatives Science. WILSON, 1958, 1343
- **WALLACE H. CAROTHERS.** *J. Am. Chem Soc.,* 1929, vol. 51 (8), 2548-59, 2560-70
- **ABHIJEET ANAND et al.** *Progress in Organic Coatings,* Août 2012, vol. 74 (4), 764-67
- **L. GUSTINI et al.** *European Polymer Journal,* Juin 2015, vol. 67, 459-75